# EUROPEAN PATENT SPECIFICATION

(11) **EP 3 887 550 B1**
(45) Date of publication and mention of the grant of the patent: **27.05.2026**
(21) Application number: 19817974.9
(22) Date of filing: 28.11.2019
(51) Int. Cl.: C12Q 1/6886, G01N 33/574, A61P 35/00

(54) **MYB-RELATED TRANSCRIPTION FACTOR (MYPOP) AS DIAGNOSTIC MARKER AND THERAPEUTIC TARGET FOR TUMOR THERAPY**
MYB-VERWANDTER TRANSKRIPTIONSFAKTOR (MYPOP) ALS DIAGNOSTISCHER MARKER UND THERAPEUTISCHES ZIELMOLEKÜL FÜR DIE KREBSTHERAPIE
FACTEUR DE TRANSCRIPTION ASSOCIÉ À MYB (MYPOP) EN TANT QUE MARQUEUR DIAGNOSTIQUE ET CIBLE THÉRAPEUTIQUE POUR UNE THÉRAPIE TUMORALE

(30) Priority: 30.11.2018 US 201816206526
(43) Date of publication of application: 06.10.2021
(73) Proprietor: Universitätsmedizin der Johannes Gutenberg-Universität Mainz, 55131 Mainz (DE)
(72) Inventor: FLORIN, Luise, 55128 Mainz (DE); WÜSTENHAGEN, Elena, 55118 Mainz (DE); SCHNEIDER, Marc, 65462 Ginsheim-Gustavsburg (DE)
(74) Representative: Engelhard, Markus
(86) International application number: PCT/EP2019/082845
(87) International publication number: WO 2020/109439

(56) References cited:
- WO-A2-2012/174282
- DEISY MORSELLI GYSI ET AL: "Comparing multiple networks using the Co-expression Differential Network Analysis (CoDiNA)", ARXIV.ORG, CORNELL UNIVERSITY LIBRARY, 201 OLIN LIBRARY CORNELL UNIVERSITY ITHACA, NY 14853, 2 February 2018 (2018-02-02), XP081409870
- WÜSTENHAGEN ELENA ET AL: "The Myb-related protein MYPOP is a novel intrinsic host restriction factor of oncogenic human papillomaviruses", ONCOGENE, NATURE PUBLISHING GROUP UK, LONDON, vol. 37, no. 48, 17 July 2018 (2018-07-17), pages 6275 - 6284, XP036647638, ISSN: 0950-9232, [retrieved on 20180717], DOI: 10.1038/S41388-018-0398-6
- ANONYMOUS: "Körpereigenes Protein unterdrückt Tumorbildung", 26 July 2018 (2018-07-26), XP055672632, Retrieved from the Internet <URL:https://www.mta-dialog.de/artikel/koerpereigenes-protein-unterdrueckt-tumorbildung.html> [retrieved on 20200302]
- M. LEDERER: "Profilin regulates the activity of p42POP, a novel Myb-related transcription factor", JOURNAL OF CELL SCIENCE, vol. 118, no. 2, 15 January 2005 (2005-01-15), pages 331 - 341, XP055673399, ISSN: 0021-9533, DOI: 10.1242/jcs.01618

## Description

### TECHNICAL FIELD

This invention relates to a method for the diagnosis or prognosis of a cancer. The invention further relates to a pharmaceutical composition, comprising a therapeutically effective amount of a Myb-related transcription factor (MYPOP) L form expressing molecule or MYPOP L form protein and a pharmaceutically acceptable carrier for use in the treatment or prevention of a tumor disease, wherein the tumor disease is selected from the group consisting of melanoma, breast cancer, lung cancer, liver cancer, gastric cancer, colon cancer, pancreatic cancer, prostate cancer, ovarian cancer, lymphoma, leukemia, kidney cancer, bladder cancer, or endometrial cancer. The invention also relates to a method of enhancing cell proliferation in a cell culture comprising the incubation of the cells in a medium that contains an inhibitor of MYPOP. The invention is set out in the appended set of claims.

### BACKGROUND ART

Myb-related transcription factor (MYPOP) is a novel protein, for which homologs have been identified in mammals, such as in human, mouse and rat. It was shown that MYPOP's orthologous murine protein p42^{POP} is able to repress the consensus Myb recognition element (MRE) when introduced into the minimal herpesvirus thymidine kinase promoter (Lederer M. Jockusch BM, Rothkegel M. Profilin regulates the activity of p42POP, a novel Myb-related transcription factor, J Cell Sci. 2005;118:331-41). It was found previously by the inventors that MYPOP acts a restriction factor for the oncogenic human papilloma virus (HPV) types 16 and 18 because it represses the long control region (LCR) activity of both viruses (Wüstenhagen et al., The Myb-related protein MYPOP is a novel intrinsic host restriction factor of oncogenic human papillomaviruses, Oncogene, 17 July 2018). It was further found by the inventors that MYPOP is eliminated in HPV-transformed tumor cells on a post-transcriptional level. As such, total MYPOP amounts were found to be strongly reduced in virus HPV-transformed cell lines in cervical cancer.

Now, the inventors found that MYPOP is also strongly down-regulated in all other tested tumor cells like melanoma, breast, hepatoma and lung cancers. This was verified in tumor samples from lung cancer patients. In these primary lung tumor cells and in other tumor cell lines, MYPOP restoration led to a block of cell proliferation. Therefore, MYPOP is a general tumor suppressor that is absent in all tumor cells and recovery of MYPOP heals cancer. Moreover, the inventors found that increased MYPOP mRNA levels, most likely a compensatory mechanism of the cancer cell after MYPOP protein loss, correlate with a poor prognosis and reduced survival rates.

The murine form of MYPOP, p42^{POP}, interacts with profilin in the nucleus (Lederer M. Jockusch BM, Rothkegel M. Profilin regulates the activity of p42POP, a novel Myb-related transcription factor, J Cell Sci. 2005;118:331-41). p42^{POP} comprises a unique combination of a single Myb-like domain, an acidic domain, nuclear import and export signals, a leucine zipper and a proline cluster mediating profilin. Amino acid sequence comparisons in databases revealed p42^{POP} to be a novel, previously non-identified protein. The N-terminus of p42^{POP} contains a tryptophan cluster motif, similar to the DNA-binding domain (DBD) of Myb-related transcription factors, followed by an acidic region, which is reminiscent of the transcriptional activation domains, present in several Myb proteins. Furthermore, p42^{POP} binds to Myb DNA consensus sequence, for which the N-terminal region that contains a Myb-related DNA binding domain is responsible (Lederer M. Jockusch BM, Rothkegel M. Profilin regulates the activity of p42POP, a novel Myb-related transcription factor, J Cell Sci. 2005;118:331-41). WO 2012/174282 A2 relates to biomarkers that can be assessed for diagnostic, therapy-related or prognostic methods. Wustenhagen et al. Oncogene (2018), 37:6275-6284, relate to MYPOP as an intrinsic host restriction factor of oncogenic human papillomaviruses. The abstract "Körpereigenes Protein unterdrückt Tumorbildung", Medizin Krebs, 26.07.2018, URL:https://www.mta-dialog.de/artikel/koerpereigenes-protein-unterdrueckt-tumorbildung.html [retrieved on 2020-03-02], relates to an antiviral effect of MYPOP. Lederer et al. (2005) JOURNAL OF CELL SCIENCE, vol. 118, no. 2, pages 331-341, describe that profilin regulates the activity of p42^{POP}, a Myb-related transcription factor.

Until now, the diagnostic and pharmaceutical properties of MYPOP / p42^{POP} have not been investigated and remained unknown.

It is still a major task of modern cancer therapy to identify novel markers for the reliable diagnosis and prognosis of cancer, and to be in possession of active agents that specifically target tumor cells but not normal, healthy cells for treating a tumor disease.

Against this background it is the object of the present invention to provide new methods and compositions for diagnosis, prognosis and therapy of a tumor disease in a human or animal patient.

This object is solved by the methods and compositions presented by the present invention. Preferred embodiments relate to specific aspects of the present invention and are claimed in the sub-claims.

### DISCLOSURE OF INVENTION

The inventors of the present invention have shown that MYPOP expression (the human equivalent / homolog to p42^{POP} found in mice) senses incoming viruses and represses viral gene transcription (Wüstenhagen et al., The Myb-related protein MYPOP is a novel intrinsic host restriction factor of oncogenic human papillomaviruses, Oncogene, 17 July 2018). Thereby, MYPOP acts as a restriction factor and limits cells' permissiveness to infection with oncogenic HPV viruses. This results in silencing of HPV early, oncogenic expression and subsequently, suppression of cancer.

The inventors surprisingly found that MYPOP is also strongly down-regulated in all other tested tumor cells like melanoma, breast, hepatoma and lung cancers. As an example, this was verified in tumor samples from lung cancer patients. In these primary lung tumor cells and in other tumor cell lines, MYPOP restoration led to a block of cell proliferation. Therefore, MYPOP has been identified as a general tumor suppressor that is absent in all tumor cells and it is evident that recovery of MYPOP heals cancer. Moreover, the inventors found that increased MYPOP mRNA levels, most likely a compensatory mechanism of the cancer cell after MYPOP protein loss, correlate with a poor prognosis and reduced survival rates of patients suffering from a cancer disease.

In the present invention, the inventors identified MYPOP as existing in two iso-forms or in two differently modified forms having a molecular weight of about 60 kDa and 42 kDa, respectively. As shown herein, the larger (L) form of MYPOP is significantly reduced or absent in tumor cells. As identified in the present invention, mRNA level negatively correlates with tumor progression, which most likely is a compensation for the loss of the MYPOP protein. This makes MYPOP as a suitable marker for the diagnosis or prognosis of cancer when analyzed on mRNA and/or protein level.

The inventive methods for diagnosing or monitoring of a cancer disease analyze the expression level of MYPOP in cells of affected tissue. The methods involve detecting and/or quantitating MYPOP protein or MYPOP mRNA in a biological sample, such as tumor tissue isolated from a human or animal patient. The methods include the steps of (i) contacting/reacting the biological sample with an antibody that is specific for MYPOP which is directly or indirectly labelled with a detectable substance and (ii) detecting the detectable substance. A reduced expression level of MYPOP protein, or an increased expression level of MYPOP mRNA in a biological sample is indicative for the presence or progression of a tumor in the subject.

As shown herein, an increase of MYPOP mRNA level negatively correlates with survival of affected tumor patients, which makes MYPOP a suitable prognostic marker to evaluate tumor progression. The determination or analysis of MYPOP mRNA can be conducted, for instance, by using cDNA derived from MYPOP mRNA followed by a quantitative polymerase chain reaction qPCR).

The methods for the diagnosis or prognosis of a cancer comprise (i) determining the level of expression of MYPOP, or parts thereof, in a patient sample, which may be a human or animal sample, (ii) comparing the determined level of MYPOP expression, or parts thereof, to the expression levels observed in a normal sample, (iii) correlating the level of MYPOP expression in the patient sample relative to the levels in the normal sample with a positive or negative diagnosis or prognosis of cancer.

A positive diagnosis of cancer is likely, if the expression level of MYPOP protein in the patient sample is reduced or if MYPOP's mRNA is overexpressed as compared to normal, non-tumor cells. A continuous reduction of MYPOP protein or an increase of the MYPOP mRNA level in the patient sample can be used to quantitatively analyze tumor progression in order to give the patient a qualitative prognosis for the further development of the cancer and hence survival. A negative diagnosis of a cancer would occur, if the expression level of MYPOP corresponds to the expression level of MYPOP found in normal tissue.

In a preferred embodiment of the invention, the larger (L) form of about 60 kDa of MYPOP protein is used as a biological indicator to determine whether a patient sample has a positive or negative diagnosis of cancer. A reduction or a lack of the 60 kDa L form of MYPOP is indicative for a positive diagnosis of cancer. A quantitative analysis of the amount of the L form of MYPOP in the biological sample allows predicting the further progression of a tumor and thus gives a prognosis of the further development of cancer in the patient. A restoration of MYPOP protein level or MYPOP protein expression (e.g. by gene therapy) in tumor cells results in reduced cell division and subsequent cell death. As such, MYPOP is a suitable therapeutic target for the treatment and/or the prevention of a cancer.

In another aspect, the invention also relates to pharmaceutical compositions comprising a therapeutically effective amount of a Myb-related transcription factor (MYPOP) L form expressing molecule or MYPOP L form protein and a pharmaceutically acceptable carrier for use in the treatment or prevention of a tumor disease. As shown herein, MYPOP reduces cell proliferation and induces cell death. As such, MYPOP can be specifically be used for antiviral and/or cancer therapy. This can be done by administering MYPOP to a patient, or by reestablishing protein expression of MYPOP in affected tumor cells.

The pharmaceutical compositions for use of the invention either contain MYPOP L form, or one or more substances that enhance MYPOP L form expression in affected tumor cells in vivo. The pharmaceutical compositions for use of the invention are for use in the treatment or prevention of a tumor disease selected from melanoma, breast cancer, lung cancer, liver cancer, gastric cancer, colon cancer, pancreatic cancer, prostate cancer, ovarian cancer, lymphoma, leukemia, kidney cancer, bladder cancer, or endometrial cancer.

The invention further relates to methods of enhancing cell proliferation in a cell culture comprising the incubation of the cells in a medium that contains an inhibitor of MYPOP expression. This makes MYPOP a suitable target for promoting the growth of cell cultures in order to increase cell mass and outcome.

### BRIEF DESCRIPTION OF DRAWINGS

- Fig. 1: illustrates that MYPOP is reduced in HPV-transformed cancer cell lines.
- Fig. 2: shows that the L form of MYPOP is strongly reduced in all tested tumor cell lines (1: primary cells, 2-4: tumor cell lines).
- Fig. 3: shows that MYPOP L form is strongly reduced in non-small-cell lung cancer (NSCLC) patients with squamous cell carcinoma (A) or adenocarcinoma (B). T = tumor, N = normal tissue.
- Fig. 4: shows that the two different iso-forms of MYPOP (L and S forms) can be differentially detected by two different antibodies.
- Fig. 5: shows that the mRNA levels of MYPOP are twofold increased in NSCLC tumor tissues compared to normal lung.
- Fig. 6: shows that the mRNA level of MYPOP in tumor and normal tissue negatively correlates with overall survival of cancer NSCLC patients.
- Fig. 7: illustrates that MYPOP inhibits colony formation of HPV-transformed and non-virally transformed cells.
- Fig. 8: shows that MYPOP re-expression reduces cell proliferation and induces cell death in breast cancer cells.
- Fig.9: shows that MYPOP re-expression reduces cell proliferation and induces cell death in NSCLC cells.
- Fig. 10: shows that MYPOP-Flag or MYPOP-N (DNA binding domain) modulate promoter activity.
- Fig. 11: shows that MYPOP-Flag or MYPOP-N (DNA binding domain) are both active in blocking cell proliferation of cancer cells.
- Fig. 12: shows that MYPOP L form is a SUMO-1 modified MYPOP protein.
- Fig. 13: shows that MYPOP protein level negatively correlates with cell proliferation.
- Fig. 14: shows that MYPOP down-regulation enhances protein expression.
- Fig. 15: shows that MYPOP re-expression reduces cell proliferation and induces cell death in liver, lung squamous and adenocarcinoma cell lines.
- Fig. 16: shows the testing of HPV16 pseudoviruses (PsVs) transduction efficiency of MYPOP and MYPOP-C PsVs by MYPOP-expression levels.
- Fig. 17: shows that MYPOP-vector (gene therapy) is functional in reducing cell proliferation of keratinocyte cancer cell lines (therapeutic target).
- Fig. 18: shows that MYPOP-vector (gene therapy) is functional in reducing cell proliferation of lung cancer cell lines (therapeutic target).
- Fig. 19: shows that MYPOP-vector has no or only a mild effect on normal human epithelial keratinocytes (NHEK).

### DETAILED DESCRIPTION OF THE INVENTION

As the technology is described herein, the section headings used are for organizational purposes only and are not to be construed as limiting the subject-matter in any way.

In this detailed description of the various embodiments, for purposes of explanation, numerous specific details are set forth to provide a thorough understanding of the embodiments disclosed. One skilled in the art will appreciate, however, that these various embodiments may be practiced with or without these specific details. Furthermore, one skilled in the art can readily appreciate that the specific steps in which methods are presented and performed are illustrative and it is contemplated that the steps can be varied and still remain within the scope of the various embodiments disclosed herein.

Unless defined otherwise, all technical and scientific terms used herein have the same meaning as is commonly understood by one of ordinary skill in the art to which the various embodiments described herein belongs. When definitions of terms in the references appear to differ from the definitions provided in the present teachings, the definition provided in the present teachings shall control.

### DEFINITIONS

To facilitate an understanding of the present technology, a number of terms and phrases are defined below. Additional definitions are set forth throughout the detailed description. Throughout the specification and claims, the following terms take the meanings explicitly associated herein, unless the context clearly dictates otherwise.

The term "MYPOP" refers to a novel Myb-related transcription factor as identified as p42^{POP} in mice (Lederer M. Jockusch BM, Rothkegel M. Profilin regulates the activity of p42POP, a novel Myb-related transcription factor, J Cell Sci. 2005;118:331-41) and in humans (Wüstenhagen et al., The Myb-related protein MYPOP is a novel intrinsic host restriction factor of oncogenic human papillomaviruses, Oncogene, 17 July 2018). For the diagnosis or prognosis it is sufficient that only a part or fragment is used for the analysis. The term "MYPOP" shall therefore also comprise truncated or elongated forms of MYPOP.

The term "L form" as used herein refers to the 60 kDa form of MYPOP as identified, for instance, by Western Blotting or other suitable methods.

The term "S form" as used herein refers to the 42 kDa form of MYPOP as identified, for instance, by Western Blotting, or other suitable methods.

The phrase "in one embodiment" as used herein does not necessarily refer to the same embodiment, though it may. Furthermore, the phrase "in another embodiment" as used herein does not necessarily refer to a different embodiment, although it may. Thus, as described below, various embodiments of the invention may be readily combined, without departing from the scope of the invention.

In addition, as used herein, the term "or" is an inclusive "or" operator and is equivalent to the term "and/or" unless the context clearly dictates otherwise. The term "based on" is not exclusive and allows for being based on additional factors not described, unless the context clearly dictates otherwise. In addition, throughout the specification, the meaning of "a", "an", and "the" include plural references. The meaning of "in" includes "in" and "on."

The term "nucleic acid" refers to a molecule having two or more deoxyribonucleotides or ribonucleotides, which may be unmodified or modified DNA or RNA. The exact size will depend on many factors, which in turn depends on the ultimate function or use of the oligonucleotide. The oligonucleotide may be generated in any manner, including chemical synthesis, DNA replication, reverse transcription, or a combination thereof. Typical deoxyribonucleotides for DNA are thymine, adenine, cytosine, and guanine. Typical ribonucleotides for RNA are uracil, adenine, cytosine, and guanine.

As used herein, the term "nucleoside" refers to a molecule having a purine or pyrimidine base covalently linked to a ribose or deoxyribose sugar. Exemplary nucleosides include adenosine, guanosine, cytidine, uridine and thymidine.

The term "nucleotide' refers to a nucleoside having one or more phosphate groups joined in ester linkages to the sugar moiety. Exemplary nucleotides include nucleoside monophosphates, diphosphates and triphosphates.

As used herein, the term "mRNA" refers generally to a single-stranded polymer of ribonucleotides. "RNA" can also refer to a polymer comprising primarily (i.e., greater than 80% or, preferably greater than 90%) ribonucleotides but optionally including at least one non-ribonucleotide molecule, for example, at least one deoxyribonucleotide and/or at least one nucleotide analog.

The term "DNA" refers generally to a polymer of deoxyribonucleotides. DNA and RNA molecules can be synthesized naturally (e.g., by DNA replication or transcription of DNA, respectively). RNA molecules can be post-transcriptionally modified. DNA and RNA molecules can also be chemically synthesized. DNA and RNA molecules can be single-stranded (i.e., ssRNA and ssDNA, respectively) or multi-stranded (e.g., double stranded, i.e. dsRNA and dsDNA, respectively).

The term "modified nucleotide" refers to a non-standard nucleotide, including non-naturally occurring ribonucleotides or deoxyribonucleotides. Preferred nucleotide analogs are modified at any position so as to alter certain chemical properties of the nucleotide yet retain the ability of the nucleotide analog to perform its intended function.

The term "degenerative" refers to a genetic code in which different codons can code for one amino acid (e.g. GUU, GUA, GUG, all coding for valine).

As used herein, the term "variant" in reference to a nucleic acid sequence refer to a nucleic acid sequence that differs by one or more nucleotides from another, usually related, nucleotide acid sequence. The term "variant" when used in reference to an amino acid sequence refer to an amino acid sequence that differs by one or more amino acids from another, usually related, amino acid sequence.

A "homolog" relates to MYPOP as found in other organisms or species.

The term "diagnosis" generally includes determination of a subject's susceptibility to a disease or disorder, determination as to whether a subject is presently affected by a disease or disorder, prognosis of a subject affected by a disease or disorder (e.g., identification of pre-metastatic or metastatic cancerous states, stages of cancer, or responsiveness of cancer to therapy), and therametrics (e.g., monitoring a subject's condition to provide information as to the effect or efficacy of therapy). A diagnosis preferably includes the detection or identification of a disease state or condition of a subject, determining the likelihood that a subject will contract a given disease or condition, determining the likelihood that a subject with a disease or condition will respond to therapy, determining the prognosis of a subject with a disease or condition (or its likely progression or regression), and determining the effect of a treatment on a subject with a disease or condition. For example, a diagnostic can be used for detecting the presence or likelihood of a subject suffering of cervical or breast cancer or the likelihood that such a subject will respond favorably to a compound (e.g., a pharmaceutical, e.g., a drug) or other treatment.

The term "marker", as used herein, refers to a substance (e.g., a nucleic acid or a region of a nucleic acid) that is able to diagnose or prognose a disorder (e.g., a cancerous disorder) by distinguishing disorder-associated cells from normal cells.

As used herein, the terms "patient" or "subject" refer to organisms to be subject to various tests or therapies provided by the invention. The term "subject" includes animals, preferably mammals, including humans.

As used herein, the terms "cancer", "tumor", and "carcinoma", are used interchangeably herein to refer to cells which exhibit relatively autonomous growth, so that they exhibit an aberrant growth phenotype characterized by a significant loss of control of cell proliferation. In general, cells of interest for detection or treatment in the present application include precancerous (e.g., benign), malignant, metastatic, and non-metastatic cells. Detection of cancerous cell is of particular interest.

A "sample" or "biological sample" or "patient sample" can be any composition of matter of interest from a human or non-human subject, in any physical state (e.g., solid, liquid, semisolid, vapor) and of any complexity. A preferred sample comprises animal cells or tissue obtained from a human or animal subject. Preferably, the sample is a biological fluid. The sample can be, for example, a cell culture or human tissue. Fluid homogenates of cellular tissues are biological fluids that may contain MYPOP for detection according to the disclosed methods. Others are fluid tissues, for example, blood or urine. The sample may be contained within a carrier, test tube, culture vessel, multi-well plate, or any other container.

As used herein, the term "antibody' refers to immunoglobulin molecules and immunologically active portions (fragments) of immunoglobulin molecules, i.e., molecules that contain an antibody combining site or paratope. The term is inclusive of monoclonal antibodies, polyclonal antibodies and fragments and single chain recombinant antibodies.

As used herein, the terms "administer", "introduce", "apply", "treat", "deliver", and grammatical variations thereof, are used interchangeably to provide MYPOP to target cells in vitro, ex vivo or in vivo, or provide genetically modified (engineered) cells harboring MYPOP in a subject.

The term "co-administration" and variations thereof refers to the administration of two or more agents simultaneously (in one or more preparations), or consecutively. For example, one or more types of genetically modified cells can be co-administered with other agents.

The term "vector" is used to refer to any molecule (e.g., nucleic acid, plasmid, or virus) used to transfer coding information (e.g., a MYPOP expressing molecule) to a host cell. The terms "expression vector" and "transcription vector" are used interchangeably to refer to a vector that is suitable for use in a host cell (e.g., a subject's cell) and contains nucleic acid sequences that direct and/or control the expression of exogenous nucleic acid sequences. Expression includes, but is not limited to, processes such as transcription, translation, and RNA splicing, if introns are present.

As used herein, the term "RNA interference" ("RNAi") refers to a selective intracellular degradation of RNA. RNAi occurs in cells naturally to remove foreign RNAs (e.g., viral RNAs). Natural RNAi proceeds via fragments cleaved from free dsRNA which direct the degradative mechanism to other similar RNA sequences. Alternatively, RNAi can be initiated by the hand of man, for example, to silence the expression of endogenous target genes, such as MYPOP.

As used herein, the term "small interfering RNA" ("siRNA") (also referred to in the art as "short interfering RNAs") refers to an RNA (or RNA analog) comprising between about 10-50 nucleotides (or nucleotide analogs) which is capable of directing or mediating RNA interference.

As used herein, the term "in vitro" has its art recognized meaning, e.g., involving purified reagents or extracts, e.g., cell extracts. The term "in vivo" also has its art recognized meaning, e.g., involving living cells in an organism, e.g., immortalized cells, primary cells, and/or cell lines, in an organism.

The term "detecting" and variations thereof includes assaying or otherwise determining the presence or absence of the target MYPOP (MYPOP encoding nucleic acid sequence or MYPOP gene product (polypeptide)), iso-forms thereof (e.g. L form of MYPOP), or combinations of agent bound targets, and the like, or assaying for, interrogating, ascertaining, establishing, or otherwise determining one or more factual characteristics of liver cancer, metastasis, stage, or similar conditions. The term encompasses diagnostic, prognostic, and monitoring applications for MYPOP and optionally other cancer biomarkers. In embodiments involving detection of MYPOP protein (as opposed to nucleic acid molecules encoding MYPOP protein), the detection method is preferably an ELISA-based method. Preferably, the detection method provides an output (i.e., readout or signal) with information concerning the presence, absence, or amount of MYPOP in a sample from a subject. For example, the output may be qualitative (e.g., "positive" or "negative"), or quantitative (e.g., a concentration such as nanograms per milliliter).

The term "therapeutically effective amount" as used herein, means that the amount of MYPOP alone or a combination with another agent that elicits the biological or medicinal response in cells (e.g., tissue(s)) that is being sought by a researcher, veterinarian, medical doctor or other clinician, which includes alleviation and/or prevention of the symptoms of the disease or disorder being treated. As used herein the language "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art.

### MODES FOR CARRYING OUT THE INVENTION

### MYPOP AS DIAGNOSTIC AND PROGNOSTIC MARKER

The inventors previously found that MYPOP is highly expressed in the epithelium and binds to the minor capsid protein L2 and the DNA of human papillomaviruses (HPV), which are the primary causative agents of cervical cancer and other tumors. The early promoter activity and early gene expression of the oncogenic HPV types 16 and 18 is potently silenced by MYPOP. Cellular MYPOP-depletion relieves the restriction of HPV16 infection, demonstrating that MYPOP acts as a restriction factor. MYPOP protein levels are significantly reduced in diverse HPV-transformed cell lines and in HPV-induced cervical cancer. Moreover, it appears that E7 stimulates MYPOP degradation. It was also found that overexpression of MYPOP blocks colony formation of HPV and non-virally transformed keratinocytes, suggesting that MYPOP exhibits tumor suppressor properties.

The inventors now found that MYPOP is also strongly down-regulated in all tested tumor cells like melanoma, breast, hepatoma and lung cancers. This was further verified in tumor samples from lung cancer patients. In these primary lung tumor cells and in other tumor cell lines, MYPOP restoration led to a block of cell proliferation. Therefore, MYPOP is a general tumor suppressor that is absent in all tumor cells and recovery of MYPOP heals cancer. Moreover, the inventors found that increased MYPOP mRNA levels, most likely a compensatory mechanism of the cancer cell after MYPOP protein loss, correlate with a poor prognosis and reduced survival rates of the patients.

The inventors also found that MYPOP exists in two iso-forms appearing at molecular weights of about 60 kDa and 42 kDa, respectively, as determined by Western Blot analyzes using samples of e.g. human keratinocytes or lung tissue. In tumor cells, the large (L) form of MYPOP is strongly reduced or eliminated. This makes the MYPOP L form suitable as a diagnostic marker for the detection of a cancer.

The inventive methods for the diagnosis or the prognosis of a cancer are characterized by a sequence of steps, comprising (i) determining the level of expression of MYPOP in a biological sample, (ii) comparing the levels to a normal sample, (iii) correlating the level of MYPOP expression in the patient sample relative to the normal sample to a positive or negative diagnosis or prognosis of cancer. A "normal sample" is a sample that is derived from non-tumor cells, i.e. healthy donor cells, of the same or other tissue.

The inventors identified for the first time a role of MYPOP, preferably the MYPOP L form, in tumor development both in HPV-infected and non-virally transformed cancer cells. It was a surprising finding that a reduced expression level of MYPOP protein in a biological sample isolated from tumor tissue of a human or animal patient is indicative for the presence of a tumor disease. As illustrated herein, MYPOP can be used as a universal marker for the detection of cancer of any type including but not limited to melanoma, breast cancer, lung cancer, liver cancer, gastric cancer, colon cancer, cervical carcinoma, pancreatic cancer, prostate cancer, ovarian cancer, lymphoma, leukemia, kidney cancer, bladder cancer, or endometrial cancer.

It was also found by the inventors that MYPOP, preferably the MYPOP L form, negatively correlates with cell proliferation and induces cell death. As mRNA level negatively correlates with survival of cancer patients, the mRNA level of MYPOP can be used to make a prognosis for further tumor development. This allows to evaluate the further progression of the tumor and to give the patient a prognosis for further survival. The underlying mechanisms why increased mRNA levels on the one hand and decreased protein levels of MYTOP on the other hand are specific for tumor cells are not fully understood but might involve a feedback mechanism that acts on MYPOP protein synthesis or translation.

In a preferred embodiment, the method of the invention determines the protein level of MYPOP in a biological sample of a human or animal patient. In a preferred aspect, the L form of MYPOP having a molecular weight of 60 kDa is determined, wherein the reduction or absence of that L form is indicative for the presence of tumor cells in the sample. If the protein expression level of MYPOP in the biological sample correlates with the protein expression level as found in a normal tissue sample of the same or different subject, this is indicative for a negative diagnosis of cancer. If the protein expression level of MYPOP is lower than the one in normal cells, this would constitute an indication for a positive diagnosis of cancer.

In a further aspect of the present invention, the mRNA expression levels can be used as an indicator for the diagnosis or prognosis of a cancer. Increased mRNA levels of MYPOP are indicative for a positive diagnosis of cancer and a reduced likelihood of survival of the patient. By quantitatively determining the amount of mRNA expression as compared to the normal levels found in normal tissue samples, tumor progression can be evaluated and the survival of a patient can be predicted. The higher the mRNA expression levels found in tumor tissue, the lower are the chances of survival of the patient.

In a preferred embodiment of the invention, the comparison of the level of protein expression of MYPOP comprises identifying a large form of MYPOP (MYPOP L form) having a molecular weight of about 60 kDa. A lack of the MYPOP L form in a patient sample is indicative for a positive diagnosis of cancer. The expression of the small form of MYPOP (MYPOP S form) having a molecular weight of 42 kDa is unaffected in tumor cells as compared to normal cells.

The determination of the level of expression of MYPOP can be executed either on mRNA level or on protein level. In a preferred embodiment, the determination of the level of expression of MYPOP protein is carried out with an antibody that is specific for MYPOP. Any polyclonal or monoclonal antibody that is specific for MYPOP can be used. In an even more preferred embodiment, the determination of the level of expression of MYPOP protein is carried out with an antibody that is specific for the N-terminus of MYPOP. Preferably, the antibody is labelled with a tag or label that confers a detectable signal. Such labels include, but are not limited to, enzymes such as alkaline phosphatase, glucose-6-phosphate dehydrogenase, and horseradish peroxidase, ribozyme, promoters, dyes, fluorescers, such as fluorescein, isothiocynate, rhodamine compounds, phycoerythrin, phycocyanin, allophycocyanin, o-phthaldehyde, and fluorescam ine, chemiluminescers such as isoluminol, sensitizers, coenzymes, enzyme substrates, radiolabels, particles such as latex or carbon particles, liposomes, cells, etc., which may be further labeled with a dye, catalyst or other detectable group.

Any biological sample can be used in the methods of the present invention, including but not limited to solid tissues or fluids, such as blood or urine. Cancer cells can be identified in any tumor tissues or fluids, such as in primary melanoma cells, cervic carcinoma cells, melanoma cells, breast cancer cells or lung carcinoma cells, because MYPOP biological activity or protein is either strongly reduced or eliminated in cancer cells.

Preferred MYPOP products are characterized by the Sequence Listing presented herein containing the sequences of SEQ ID NO: 1 to SEQ ID NO: 4.

In a preferred embodiment, the MYPOP protein is encoded by a nucleic acid sequence that comprises the mRNA sequence of SEQ ID NO: 1:The invention also comprises variants or RNA analogs having at least one altered or modified nucleotide as compared to the corresponding unaltered or unmodified mRNA.

The amino acid sequence of SEQ ID NO: 2 relates to the N-terminus of MYPOP, and includes the first 126 amino acids containing the DNA binding domain and a single nuclear localisation signal (NLS). The amino acid sequence of SEQ ID NO: 3 relates to the full length amino acid sequence of MYPOP in humans, the amino acid sequence of SEQ ID NO: 4 relates to the full length amino acid sequences of MYPOP in mice, and the amino acid sequence of SEQ ID NO: 5 relates to the full length amino acid sequences of MYPOP in rats. The invention however also covers any other protein or nucleic acid that exhibits a degree of homology to MYPOP found in other organisms. Preferably, such a homolog has at least 50 %, preferably at least 60 %, more preferably at least 70 %, more preferably at least 80 %, more preferably at least 90 %, and even more preferably at least 95 % homology with the MYPOP amino acid sequence set forth in SEQ ID NO: 2. As such, the nucleic acid sequences according to SEQ ID NO: 1 or the amino acid sequences of SEQ ID NO: 2 - 4 may include variations (substitutions, additions, deletions) without significantly changing the biological behaviour and/or activity of the encoded MYPOP gene product.

The MYPOP preferably comprises a nucleic acid sequence that encodes the amino acid sequence of MYPOP or a variant thereof in which one or more nucleic acids are substituted by degenerate nucleic acids resulting in the same amino acid sequence of MYPOP. In a preferred embodiment, the invention also covers vectors or plasmids that contain the gene of MYPOP for transforming and expressing MYPOP in tumor cells. In an even more preferred embodiment, the invention also covers vectors or plasmids that contain the gene of MYPOP encoding for the DNA binding domain (i.e. the N-terminus of MYPOP) for transforming and expressing MYPOP in tumor cells, which shows full tumor suppressive function when compared to full length MYPOP. The encoding DNA sequence is composed of the amino acid sequence of SEQ ID NO: 2. The invention also includes nucleotide analogs, altered nucleotides or modified nucleotides of MYPOP that relate to non-naturally occurring ribonucleotides or deoxyribonucleotides.

In a preferred embodiment, MYPOP that is determined in the patient sample (e.g. biological tissue sample) comprises a nucleic acid sequence of SEQ ID NO: 1 or a homolog or variant thereof sharing at least 75 %, at least 85 %, at least 90 % or at least 95 % homology with the nucleic acid sequence of SEQ ID NO: 1 while the homolog or variant retains the biological activity of MYPOP as Myb-related transcription factor.

In a preferred embodiment, MYPOP that is determined in the patient sample (e.g. biological tissue sample) comprises an amino acid sequence of SEQ ID NO: 2, SEQ ID NO: 3 or SEQ ID NO: 4, or a homolog or variant thereof sharing at least 75 %, at least 85 %, at least 90 % or at least 95 % homology with the amino acid sequence of SEQ ID NO: 2, SEQ ID NO: 3 or SEQ ID NO: 4, while the homolog or variant retains the biological activity of MYPOP as Myb-related transcription factor.

Preferably, the determination of the level of expression of MYPOP is carried out by quantitatively or qualitatively analyzing the mRNA level or protein level of MYPOP using any one of the sequences according to SEQ ID NO: 1 to 5, or fragments thereof.

A homolog in this context refers to a protein or nucleic acid of MYPOP as found in other species, whereas a variant may include one or more nucleic acid or amino acid exchanges without altering the biological nature of MYPOP.

The determination whether MYPOP acts as a Myb-related transcription factor can be analyzed by any suitable assay known in the art, including but limited to DNA binding assay, promoter assays, Western Blot assay, or ELISA assay.

### MYPOP FOR USE IN TUMOR THERAPY

The inventor's finding that a restoration of MYPOP expression (either by gene therapy or chemotherapy) results in reduced growth of tumor cells makes MYPOP a suitable target for therapeutic agents that restore MYPOP activity in tumor cells.

A further aspect of the invention thus relates to a pharmaceutical composition, comprising a therapeutically effective amount of a Myb-related transcription factor (MYPOP) L form expressing molecule or MYPOP L form protein and a pharmaceutically acceptable carrier for use in the treatment or prevention of a tumor disease, wherein the tumor disease is selected from the group consisting of melanoma, breast cancer, lung cancer, liver cancer, gastric cancer, colon cancer, pancreatic cancer, prostate cancer, ovarian cancer, lymphoma, leukemia, kidney cancer, bladder cancer, or endometrial cancer. Disclosed herein is the use of MYPOP as suitable target in a method of treating or preventing a tumor disease. As illustrated herein, tumor progression can be reduced or fully stopped by re-restoration of the expression of MYPOP in affected tumor cells. Expressing MYPOP can be achieved by delivering to tumor cells a MYPOP-expressing viral or non-viral vehicle, plamid or vector. Alternatively, MYPOP can be administered in a suitable way to the patient, such as by oral administration, intravenous administration or by injection in affected tumor tissue. MYPOP expression can also be restored by administering suitable substances that restore MYPOP expression in tumor cells. In addition, expression or transcription vectors can be used for expressing MYPOP in affected tumor cells.

Methods of treatment of the present disclosure further include prophylactic and therapeutic methods of treating a subject at risk of (or susceptible to) a disorder or having a disorder associated with reduced MYPOP activity. Alternatively, the target gene expression or activity may be normal (non-aberrant) but an increase in MYPOP gene expression or activity would nonetheless have a beneficial effect on the subject. The references to methods of treatment of this disclosure are to be interpreted as references to the pharmaceutical composition for use of the present invention for use in such methods.

In another aspect of the present disclosure, the subject is administered an agent which modulates MYPOP expression or that accelerates MYPOP protein synthesis in affected tumor cells. Subjects at risk for a condition which is caused or contributed to by aberrant MYPOP gene expression or MYPOP mRNA overexpression, such as breast, lung or liver cancer, can be identified by, for example, any or a combination of diagnostic or prognostic assays as is known in the art and described herein.

In a preferred embodiment, a therapeutic agent inhibits one or more of the biological activities of MYPOP inactivating or degrading enzymes or restores MYPOP protein synthesis in tumor cells. Examples of such therapeutic agents include blocking peptides, small molecule inhibitors and anti-target antibodies. These modulatory methods can be performed in vitro (e.g., by culturing the cell with the agent) or, alternatively, in vivo (e.g., by administering the agent to a subject). As such, methods of treating an individual afflicted with a disease or disorder characterized by aberrant expression or activity of the target gene protein or nucleic acid molecule are disclosed herein. In one aspect of the present disclosure, the method of the present disclosure involves administering an agent, or combination of agents that modulates (e.g., upregulates or downregulates) the target gene expression or activity. In another aspect of the present disclosure, the method involves administering the target gene protein or nucleic acid molecule as therapy to compensate for reduced or aberrant target gene expression or activity. It is provided that such modulatory methods or agents result in an increase of MYPOP's biological activity or stability in affected tumor cells.

Methods of treatment of the present disclosure also include the administration or co-administration of MYPOP or a homolog thereof in order to reduce or prevent tumor progression. For example, in one aspect of the present disclosure, the method of the present disclosure involves administering a desired drug to an individual with a cell population expressing low MYPOP protein levels, and co-administering an inhibitor of target gene synthesis to modulate mRNA overexpression in tumor cells. The administration and co-administration steps can be carried out concurrently or in any order, and can be separated by a time interval sufficient to allow uptake of either compound by the cells to be eradicated. For example, a pharmaceutical composition comprising peptides or small molecules that restore MYPOP synthesis can be administered to the individual sufficiently in advance of administration of the drug to modulate the MYPOP gene expression and/or protein production. Restoration of the MYPOP activity is desirable in situations in which the MYPOP gene product is abnormally downregulated such as in cancer cells.

In a preferred embodiment, a part of MYPOP protein is administered as polypeptide, protein or peptide that preferably comprises the DNA binding domain of MYPOP, preferably comprises the N-terminus of MYPOP. In a preferred embodiment the part of MYPOP that comprises the N-terminus comprises the amino acid sequence as defined in SEQ ID NO: 2.

The dosage ranges for the administration of the therapeutic agents as disclosed herein are those large enough to produce the desired effect in which the symptoms of MYPOP are treated. The dosage should not be so large as to cause adverse side effects, such as unwanted cross-reactions, anaphylactic reactions, and the like. Generally, the dosage will vary with the age, condition, sex and extent of the symptoms in the patient and can be determined by one of skill in the art. The dosage can be adjusted by the individual physician in the event of any counter indications. The dosage amount may depend on the specific disorder and patient that are treated and can be readily determined using known dosage adjustment techniques by a physician having ordinary skill in treatment of these disorders. The dosage amount will generally lie within an established therapeutic window for the therapeutic compound which will provide a therapeutic effect while minimizing additional morbidity and mortality.

In one aspect of the treatment method of the present disclosure, it is sufficient to express the N-terminus of MYPOP containing the active domains to inhibit the progression of cancer and sensitize the tumor cells to therapeutic drugs or gene therapy, thus improving the efficacy of these treatments.

In a preferred embodiment, the MYPOP molecule is used as a target for molecular therapy. For this, several approaches, such as the application of small interfering RNA (siRNA) as MYPOP mRNA antagonists, can be used to upregulate the expression of MYPOP in tumor cells. In addition, an overexpression of MYPOP can render tumor cells more sensitive to current therapeutic drugs or gene therapy, improving the efficacy of the treatments.

It appears that MYPOP protein has a protective effect and is only reduced in tumor cells, but not in normal cells. Restoration of the protein levels of MYPOP results in a cell death or in a blockage of tumor cell divisions. As such, a preferred method of treating or preventing a tumor disease of the present disclosure is by administering MYPOP protein to a subject at risk. In a further preferred embodiment, tumor cells are transformed in a way that protein synthesis of MYPOP is maintained or increased.

The pharmaceutical composition for use of the invention is for use in the treatment or prevention of a tumor disease that can be treated by addition or restoration of MYPOP protein expression selected from the group consisting of melanoma, breast cancer, lung cancer, liver cancer, gastric cancer, colon cancer, pancreatic cancer, prostate cancer, ovarian cancer, lymphoma, leukemia, kidney cancer, bladder cancer, or endometrial cancer. However, the invention may be beneficial to other tumor diseases.

The present invention also relates to pharmaceutical compositions, comprising a therapeutically effective amount of a MYPOP L form expressing molecule or protein and a pharmaceutically acceptable carrier, for use in the treatment or prevention of a tumor disease, wherein the tumor disease is selected from the group consisting of melanoma, breast cancer, lung cancer, liver cancer, gastric cancer, colon cancer, pancreatic cancer, prostate cancer, ovarian cancer, lymphoma, leukemia, kidney cancer, bladder cancer, or endometrial cancer. Except insofar as any conventional media or agent is incompatible with the active compound, use thereof in the compositions is contemplated. Supplementary active compounds can also be incorporated into the compositions.

The pharmaceutical composition is formulated to be compatible with its intended route of administration. Examples of routes of administration include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

In one embodiment, MYPOP or agents that mediate MYPOP expression are prepared with carriers that will protect against rapid elimination from, or degradation in, the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Such formulations can be prepared using standard techniques. Liposomal suspensions (including liposomes targeted to antigen-presenting cells with monoclonal antibodies) can also be used as pharmaceutically acceptable carriers.

In another embodiment, the MYPOP gene can be inserted into genetic constructs, e.g., viral vectors, retroviral vectors, expression cassettes, or plasmid viral vectors, e.g., using methods known in the art. Genetic constructs can be delivered to a subject by, for example, inhalation, orally, intravenous injection, local administration or by stereotactic injection. The pharmaceutical preparation of the delivery vector can include the vector in an acceptable diluent, or can comprise a slow release matrix in which the delivery vehicle is imbedded. Alternatively, where the complete delivery vector can be produced intact from recombinant cells, e.g., retroviral vectors, the pharmaceutical preparation can include one or more cells which produce the polynucleotide delivery system.

### (i) Injectable Compositions

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL (BASF; Parsippany, N.J.) or phosphate buffered saline (PBS). In all cases, the composition must be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

### (ii) Oral Compositions

Oral compositions generally include an inert diluent or an edible carrier. They can be enclosed in gelatin capsules or compressed into tablets. For the purpose of oral therapeutic administration, the MYPOP compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, primogel, or corn starch; a lubricant such as magnesium stearate or sterotes; a glidant such as colloidal silicon dioxide; a sweetening agent such as sucrose or saccharin; or a flavoring agent such as peppermint, methyl salicylate, or orange flavoring. These are not typically used for administration of polynucleotides or antibodies.

### (iii) Compositions for Pulmonary Administration

For administration by inhalation, the compounds are delivered in the form of an aerosol spray from pressured container or dispenser which contains a suitable propellant, e.g., a gas such as carbon dioxide, or a nebulizer.

### (iv) Compositions for Topical Administration

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

### (v) Parenteral Administration

Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like.

The compounds can also be prepared in the form of suppositories (e.g., with conventional suppository bases such as cocoa butter and other glycerides) or retention enemas for rectal delivery.

It is especially advantageous to formulate compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms are dictated by and directly dependent on the unique characteristics of the active compound and the particular therapeutic effect to be achieved, and the limitations inherent in the art of compounding such an active compound for the treatment of individuals.

The pharmaceutical compositions can be included in a container, pack, or dispenser together with instructions for administration.

### MYPOP AS PROMOTER IN CELL CULTURES

The present invention further relates to methods of enhancing cell proliferation in a cell culture, which makes MYPOP a suitable target for enhancing cell growth, in particular the growth of productive cells, preferably productive cells of a productive cell line. For example, this can be achieved by using an inhibitor of MYPOP activity in order to increase the expression rate and/or the proliferation rate of cells. This makes MYPOP suitable for industrial applications, e.g. for production cell lines like Chinese hamster ovary (CHO) cells, yeast or other cells. A preferred inhibitor of MYPOP activity is an antibody, either monoclonal or polyclonal antibody. Alternatively, profilin can be used to inhibit MYPOP activity. By inhibiting MYPOP, the growth of cell cultures and be promoted in order to increase cell mass and outcome.

The inhibitor can be any one that inhibits MYPOP expression or MYPOP biological activity in vitro or in vivo. In a preferred embodiment, the inhibitor of MYPOP is a knockdown or knockout MYPOP gene which has been introduced into transformed cells. The expression level of MYPOP can either be reduced by inhibiting protein synthesis of MYPOP or by degrading MYPOP protein e.g. by proteases. Alternatively, RNAi or siRNA approaches can be used for selective intracelluar degradation of MYPOP RNA or for mediating RNA interference. Moreover, MYPOP knockout by e.g. CRISPR CAS9 technology can be used to inhibit MYPOP expression.

Further, expression of MYPOP can be inhibited by bringing into contact said production cell line with at least a gene editing nuclease, such as CAS9 and a guide nucleic acid, such as a guide RNA (gRNA), specific for MYPOP.

### EXAMPLES

The invention will be further illustrated in the following examples. However, the invention shall not be restricted to these examples by any form.

### (i) Example 1: MYPOP as diagnostic and prognostic marker

Experiments were conducted to show that MYPOP is strongly reduced in all tested tumor cell lines and patients. In particular, it was found by the inventors that the MYPOP L form (at about 60 kDa) is reduced in tumor cell lines.

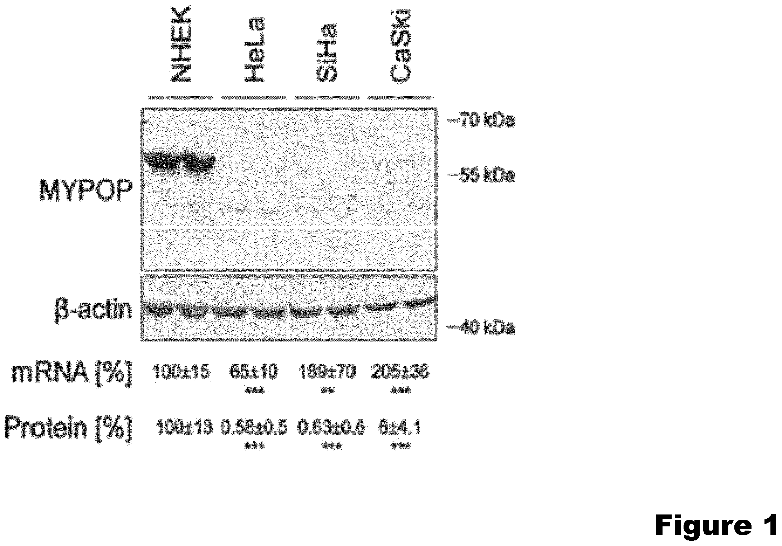

Figure 1 shows MYPOP reduction in HPV-transformed cell lines and cancer tissue. Quantification of MYPOP protein and mRNA in primary keratinocytes (NHEK) and HPV-transformed cells lines HeLa (HPV18), SiHa and CaSki (both HPV16). Total cellular mRNA was analyzed by quantitative real-time PCR (qPCR). NHEK were set to 100% and data (n=6) were analyzed using two-tailed unpaired t-test: p=0.000944, t=-4.6245, dF=10 (HeLa) or Welch two-tailed t-test p=0.01839, t=-3.3236, dF=5.4495 (SiHa) or two-tailed unpaired t-test p=6.653x10-5, t=6.5284, dF=10 (CaSki). Densitometric quantification of the Western blots (a representative Western Blot is shown in the upper panel) was performed with ImageJ software and relative MYPOP band intensities were normalized to β-actin. NHEK cells were set to 100% and data (n=5) were analyzed using Welch two-tailed t-test p=9.81x10-7, t=-19.968, dF=6.0255 (HeLa), p=9.71x10-7, t=19.949, dF=6.035 (SiHa), p=92.11x10-7, t=-17.647, dF=7.5346 (CaSki).

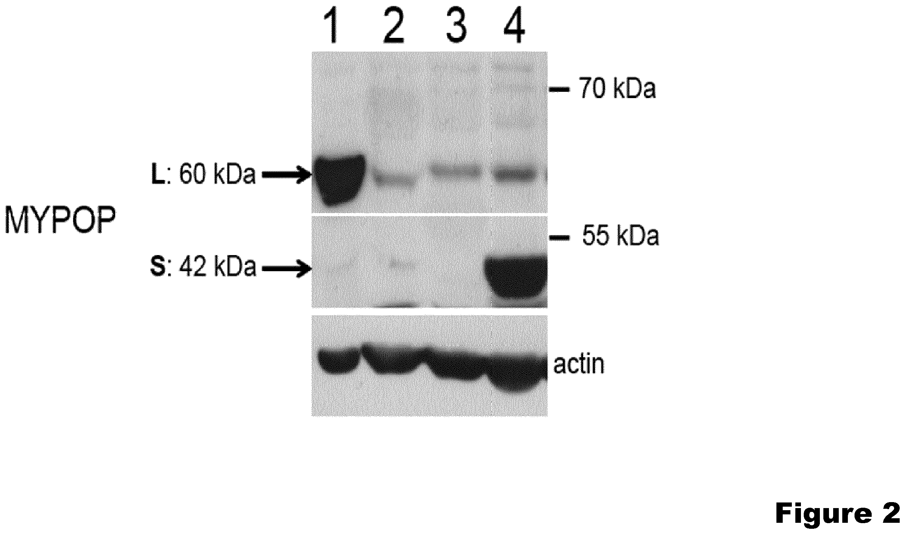

Figure 2 shows that MYPOP L form is strongly reduced in all tested tumor cell lines (cervical, melanoma, breast cancer cell lines). Western Blot shows that MYPOP exists in two forms of about 60 (large, L) and 42 kDa (small, S). The MYPOP L form is strongly reduced in cancer cell lines. 1: normal human epithelial keratinocytes (NHEK), 2: cervical carcinoma cells (SiHa), 3: melanoma cells (WM266.4), 4: breast cancer cells (MCF-7). Normal keratinocytes (NHEK) served as control and display high expression levels of MYPOP.

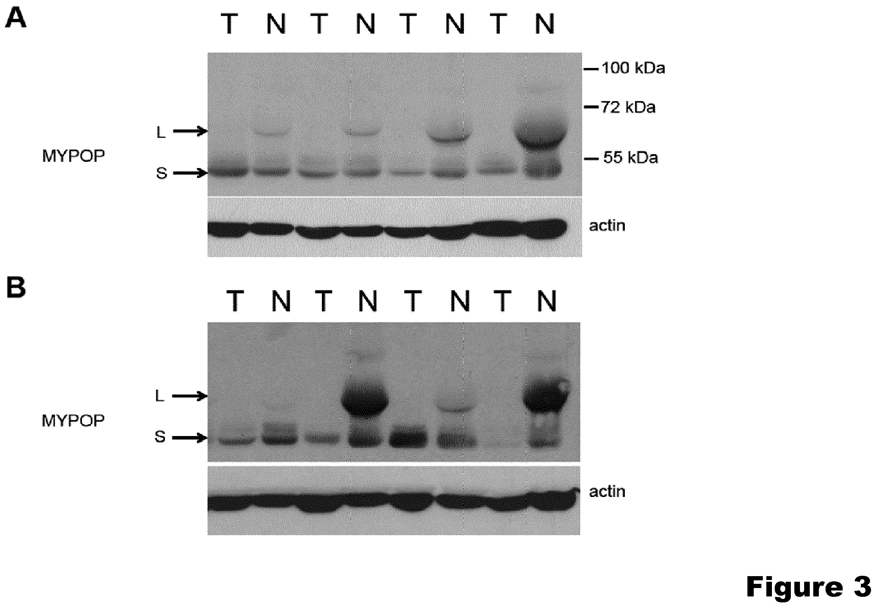

Figure 3 illustrates that the MYPOP L form is strongly reduced in all tested non-small-cell lung cancer patients with squamous cell carcinoma (A) or adenocarcinoma (B). Shown is MYPOP in Western Blot analyses of 4 patients each.

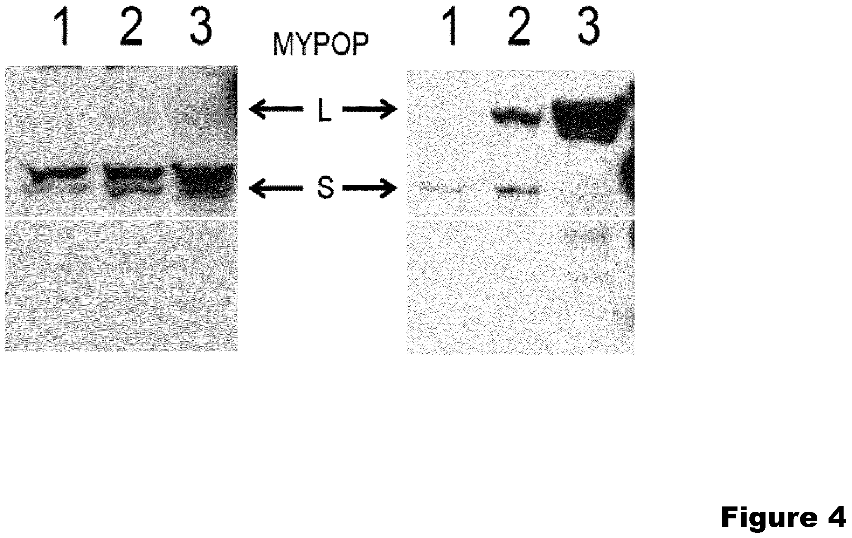

Figure 4 shows that MYPOP L form and S form can be differentially detected by two different antibodies. Shown is MYPOP in Western Blot analyzes of HeLa (1), HaCaT (2) and NHEK (3) cell lysates using polyclonal rabbit ProteoGenix antibody (left) or polyclonal rabbit Abcam antibody (right). A comparison of both MYPOP antibodies and their reactivity in immunoblot was made. HeLa, HaCaT and primary keratinocytes cell lysates were separated by SDS-PAGE, processed by Western blot and analyzed by immunodetection. Detection of MYPOP was performed using either ProteoGenix or Abcam antibody. The arrows indicate the L and S form. Due to clarity and conciseness the Western blot images are cropped.

### (ii) Example 2: MYPOP mRNA is enhanced in lung cancer cells (diagnostic marker) and expression negatively correlates with survival (prognostic marker).

MYPOP mRNA is enhanced in lung cancer cells and correlates with survival. The inventors have found that MYPOP RNA expression levels are increased in cancer cells, which makes MYPOP RNA suitable as a diagnostic marker. In addition, it was found that on the protein level MYPOP protein expression is reduced in cancer cells. It was further found that expression of MYPOP mRNA negatively correlates with survival, which makes MYPOP suitable as a prognostic marker.

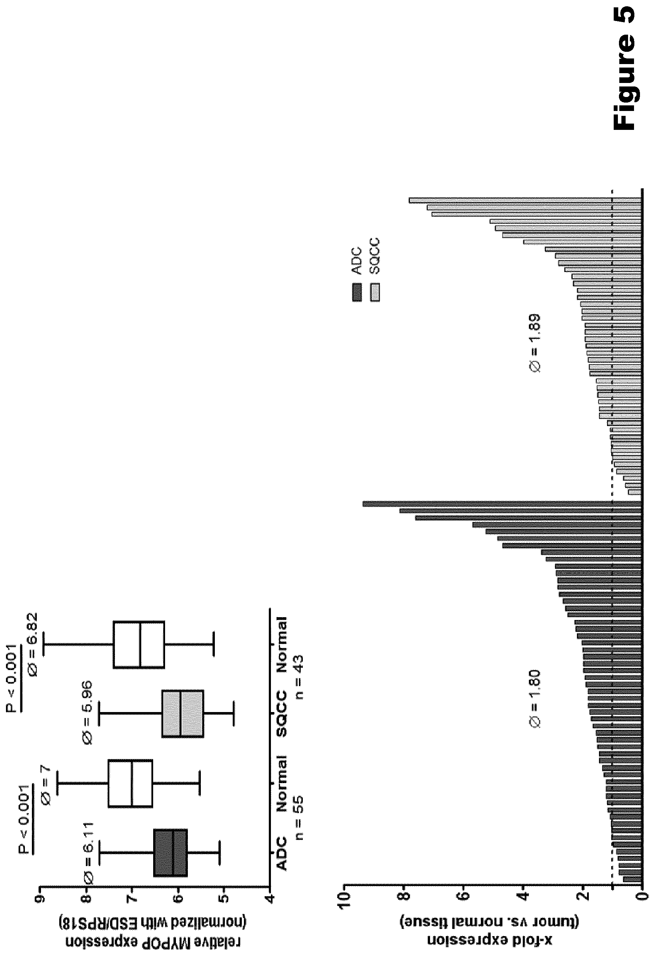

In Figure 5 it is shown that the mRNA levels of MYPOP are significant higher in tumor tissues (adenocarcinoma (n = 55) and squamous cell carcinoma (n = 43) compared to normal lung tissue (lower ΔCt-Values of MYPOP indicate a higher expression). A paired analysis shows a 2-fold increased mRNA expression in lung cancer patients (tumor vs. normal tissue). (ADC = adenocarcinoma; SQCC = squamous cell carcinoma)

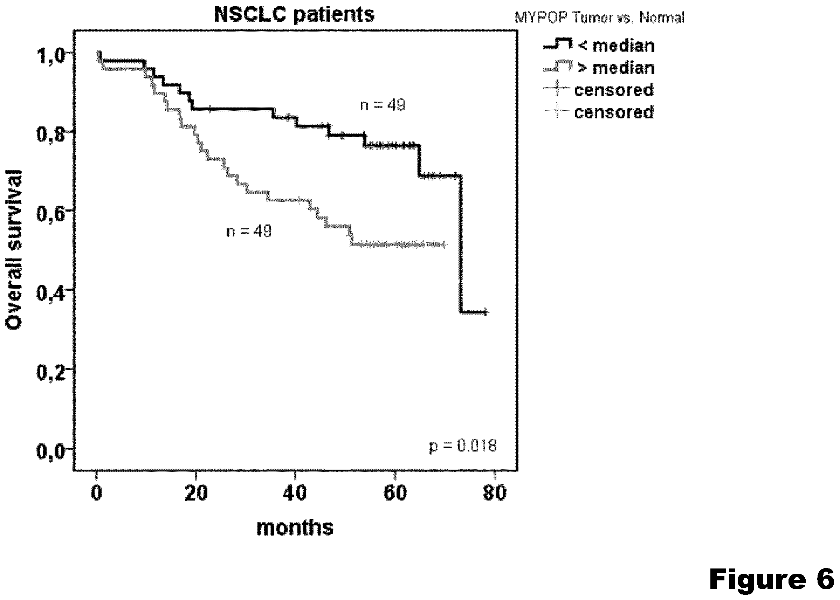

In Figure 6: MYPOP mRNA level is a prognostic marker for the survival of cancer patients. A Kaplan-Meier analysis shows that the mRNA level negatively correlates with overall survival of NSCLC patients.

The two groups were separated using the median of the fold change (tumor versus normal tissue) (median as a limit for the two groups) of all patients.
0 = low Expression
1 = high Expression
P = 0,018 => significant Influence

Strong overexpression in the tumor compared to normal tissue reduces overall survival of the patients.

### (iii) Example 3: MYPOP as therapeutic target

MYPOP reduces cell proliferation and induces cell death.

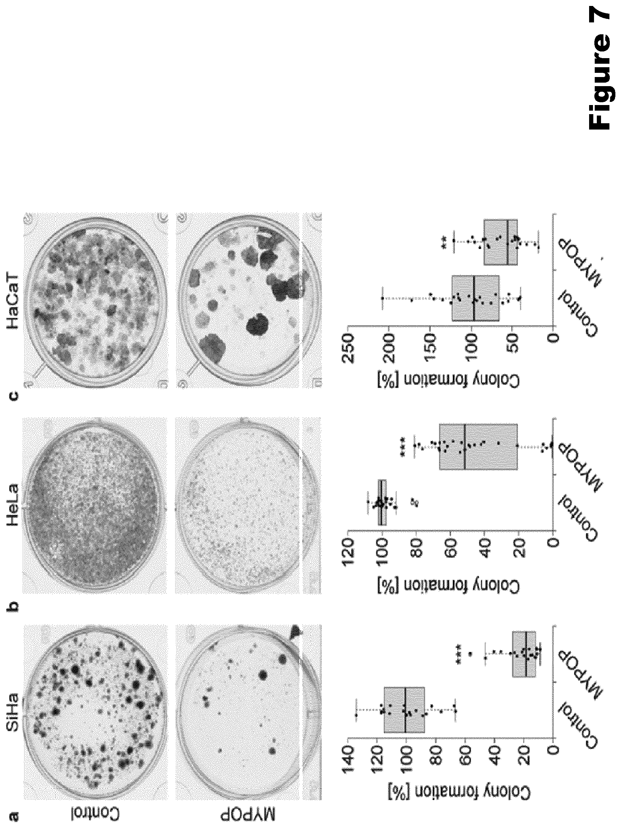

Figure 7 shows that MYPOP inhibits colony formation of HPV-transformed and non-virally transformed cells. a-c Cells were transfected with either MYPOP expression plasmid or a control plasmid and selected for 6-12 days with G418. Colonies of control or MYPOP transfected cells were fixed with methanol and stained using crystal violet (upper panel a-c). Plates were quantified using ImageJ plugin "ColonyArea" (lower panel a-c) and values are given as boxplots. A: Shown are representative image of SiHa cells and the values obtained from five independent experiments. Control-transfected cells were set to 100%. Data (n=20) were analyzed using Wilcoxon rank sum test p=1.451x10-11, W=400. B: Shown are representative image of HeLa cells and the values obtained from nine independent experiments. Control-transfected cells were set to 100%. Data (n=30) were analyzed using Wilcoxon rank sum test p=2.2x10-16, W=899. C: Shown are representative image of HaCaT cells and the values obtained from six independent experiments. Control-transfected cells were set to 100%. Data (n=22) were analyzed using Welch two-tailed t-test p=0.00105, t=3.5361, dF=39.71; **, p≤0.01, ***, p≤0.001

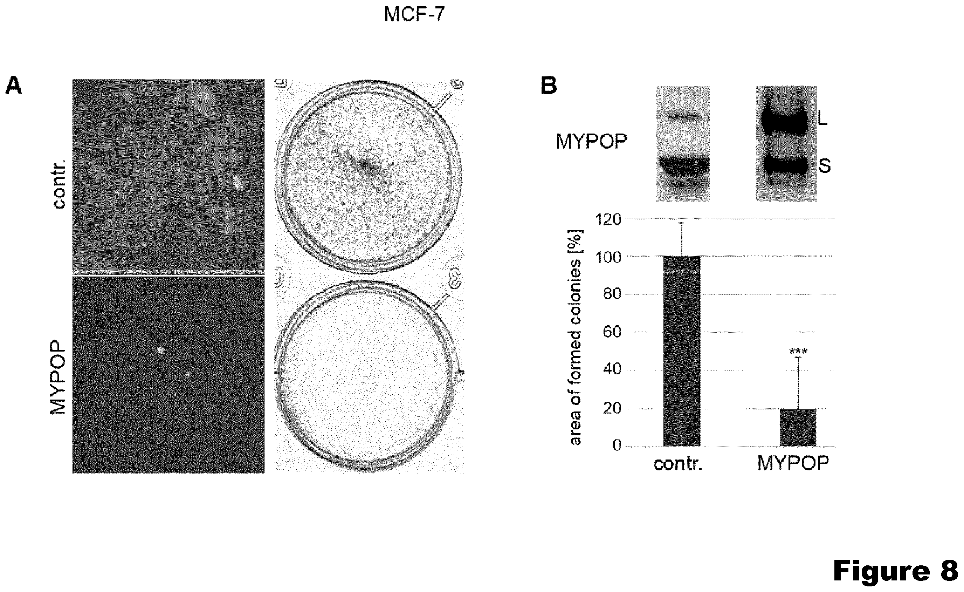

Figure 8 shows that MYPOP re-expression (restoration of the L form) reduces cell proliferation and induces cell death in breast cancer cells (MCF-7). A: MCF-7 cells were transfected with either MYPOP-GFP expression plasmid or a control GFP plasmid and selected for 10 days with G418. Shown are formed colonies of GFP or MYPOP-GFP expressing cells (left). Colonies of control or MYPOP transfected cells were fixed with methanol and stained using crystal violet (A, right). B: Western blot shows re-storage of the L-form after 24h hours of MYPOP-GFP or control transfection of cells (upper panel). Formed colonies were quantified using ImageJ plugin "ColonyArea" (lower panel). The values were obtained from two independent experiments. Control-transfected cells were set to 100%. (n=5)

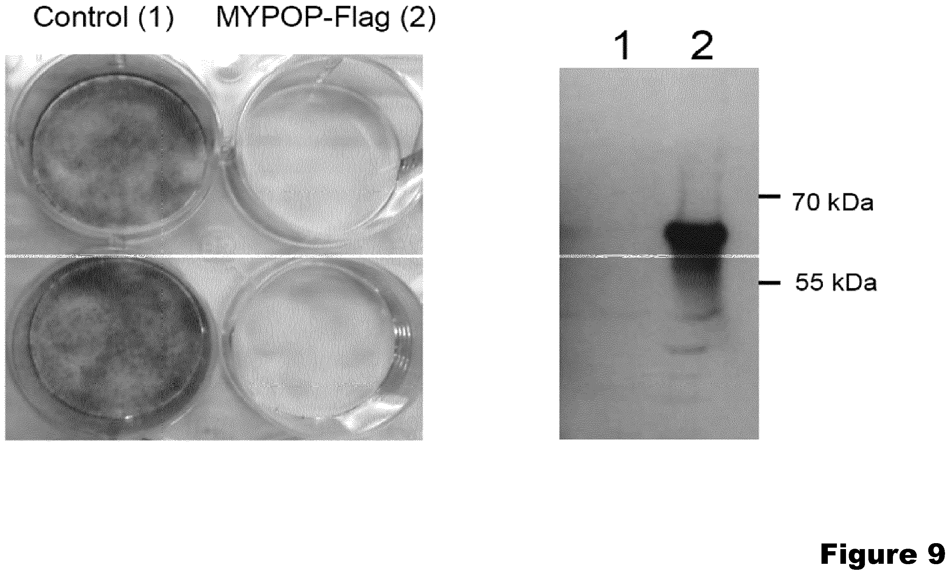

Figure 9 shows that MYPOP re-expression reduces cell proliferation and induces cell death in lung cancer cell lines isolated from a lung cancer patient. Cells were transfected with either MYPOP-Flag expression plasmid or a control Flag plasmid and selected for 7 days with G418. Shown are formed colonies of Flag (control) or MYPOP-Flag expressing cells (left). Colonies of control or MYPOP transfected cells were fixed with methanol and stained using crystal violet (left). Western blot shows re-storage of MYPOP after 24h hours of MYPOP-Flag or control transfection of cells (right).

(iv) Example 4: Cancer therapy using MYPOP as target is based on the restoration of the activity of MYPOP. In particular, it is the aim of a MYPOP-based cancer therapy to restore the activity of the MYPOP main band (L form).

In the experiments that were conducted in Figures 7 to 9 and 11, MYPOP, MYPOP-GFP, MYPOP-GST, MYPOP-His (not shown), MYPOP-Flag and MYPOP-N-Flag can be used for gene therapy of cancer patients.

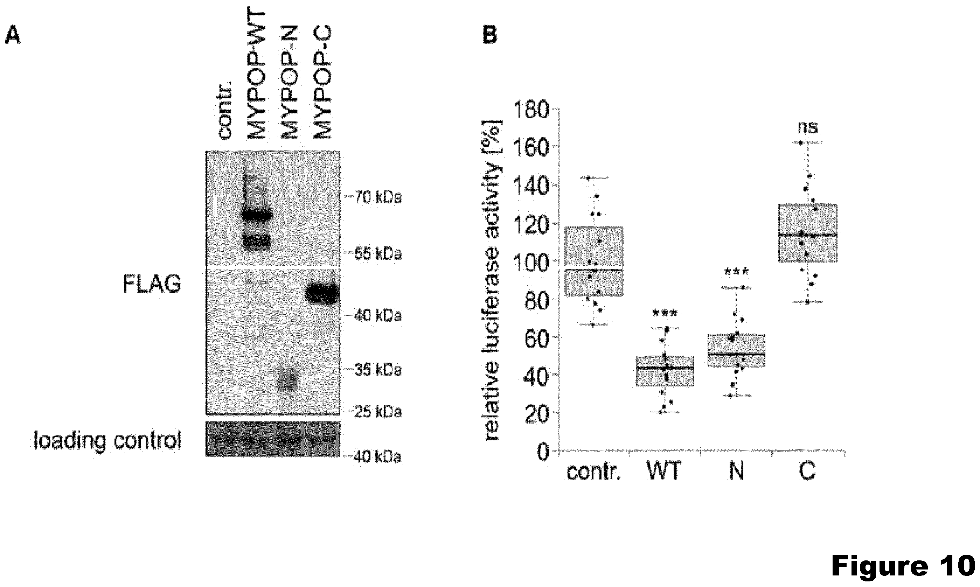

Figure 10 shows that MYPOP-Flag or MYPOP-N (containing the DNA binding domain) modulate promotor activity. (A) HaCaT cells were transiently cotransfected with pGL4.20 HPV16 LCR luciferase reporter plasmid together with control FLAG vector or MYPOP-wt, -N or -C as indicated for 24 hours. Cells were then lysed to monitor MYPOP expression by immunodetection, using monoclonal mouse FLAG (M2) antibody. The lower panel shows an unspecific band as loading control. (B) Experiment was performed as described in (A), but cells were lysed and luciferase activity, as a measure of promoter activity, was assessed and normalized by LDH measurement. The values obtained from four independent experiments are given as boxplots and pGL4.20 16 LCR with an empty FLAG vector was set to 100%. Data (n=15) were analyzed using a two-tailed unpaired t-test.

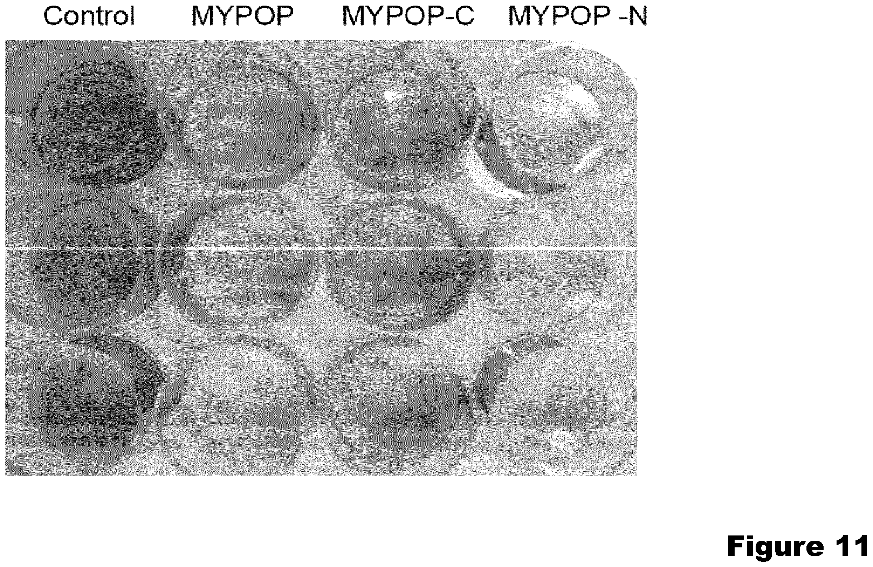

Figure 11 shows that MYPOP and MYPOP-N expression reduces cell proliferation in cancer cells. HeLa cells were transfected with either FLAG vector or MYPOP-wt, -C or - N as indicated and selected for 10 days with G418. Shown are formed colonies of Flag (control) or MYPOP-wt, -C or -N Flag expressing cells. Colonies of control or MYPOP transfected cells were fixed with methanol and stained using crystal violet.

It was further anticipated that agents that are able to stabilize the MYPOP L form are useful drugs for cancer therapy. In order to analyse the differences between the L and the S form, putative SUMOylation of MYPOP was tested. Indeed, MYPOP was modified by SUMO-1. SUMO antibody detects the MYPOP L form in purified MYPOP-Myc-his6 samples. Therefore, blocking of the desumoylation machinery or inhibition of degradation of the SUMO-modified MYPOP is expected to inhibit loss of the L form of MYPOP and prevents carcinogenesis.

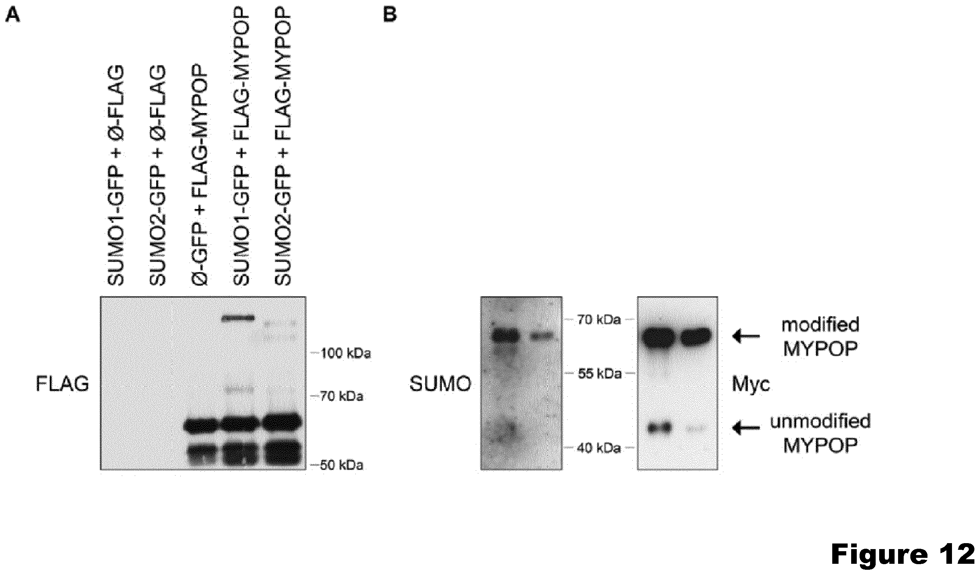

In Figure 12, MYPO L form is a SUMO-1 modified MYPOP protein. It is likely that destabilisation of MYPOP is mediated by deSUMOylation: (A) FLAG-MYPOP or Ø-FLAG was co-expressed with SUMO1-GFP, SUMO2-GFP or Ø-GFP for 24 hours in HaCaT cells. Cells were lysed and processed by SDS-PAGE and Western blot using monoclonal mouse FLAG antibody. (B) MYPOP-Myc-his6 was expressed in HEK293T cells, purified via its his6 tag and lysed in SDS sample buffer. One sample was loaded two times on a SDS-PAGE, separated and process by Western blot. The membrane was cut and each part was incubated simultaneously with either polyclonal rabbit SUMO antibody or monoclonal mouse Myc antibody.

### (v) Example 5: MYPOP for enhancing productivity

The qualities of MYPOP can be used to enhance productivity of producing cell lines. A reduction of MYPOP protein enhances proliferation and expression of cells and can thereby be used in order to accelerate cell division and cell proliferation of production cell lines.

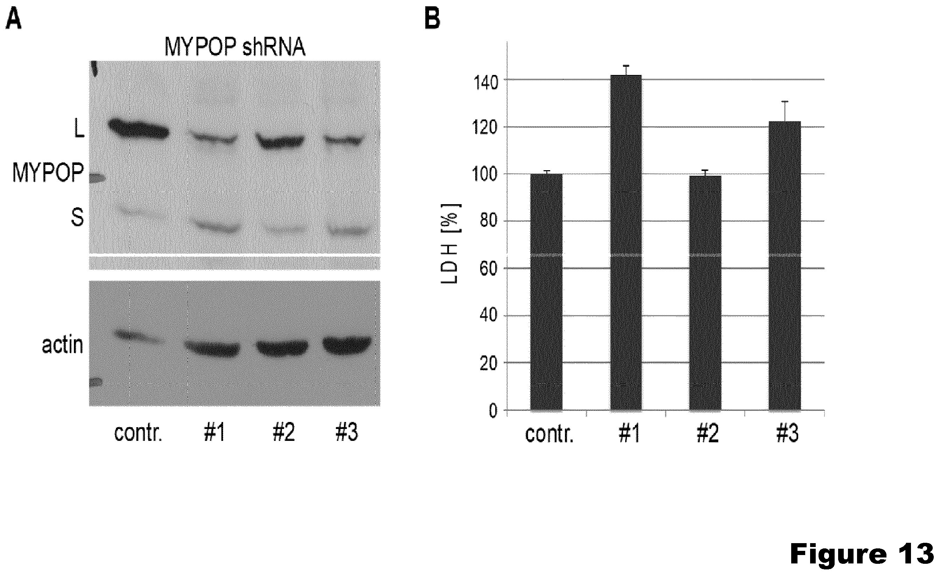

Figure 13 shows that MYPOP protein level negatively correlates with cell proliferation. This is not only useful for diagnostic or therapeutic applications, but also for industrial application to enhance the productivity of cell lines. HaCaT cells were transduced with lentiviruses containing MYPOP-specific shRNAs. Knockdown efficiency (A, upper panel), actin levels (A, lower panel), and relative LDH levels are shown. Control shRNA-treated cells were set to 100%. Actin and LDH levels serve as a measure of the amount of cells.

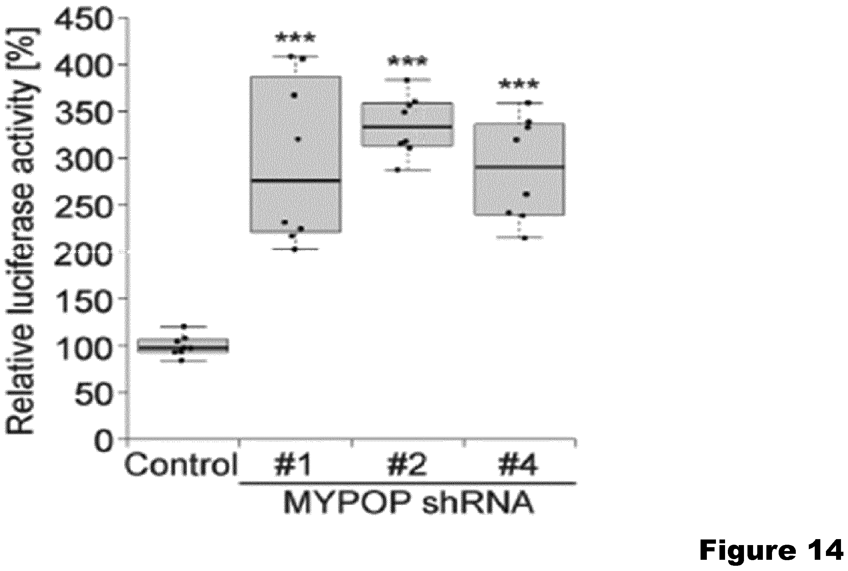

Figure 14 shows that a downregulation of MYPOP enhances protein expression. HaCaT cells were transduced with lentiviruses containing MYPOP-specific shRNAs to reduce the MYPOP protein levels. Seven days later, the cells were transduced with a luciferase vector. Relative luciferase activity as measure for gene expression was assessed 24 hours later. Control siRNA-treated cells were set to 100% and data (n=16) were analyzed using Wilcoxon rank sum test: p=0.0004666, W=39 (#9).

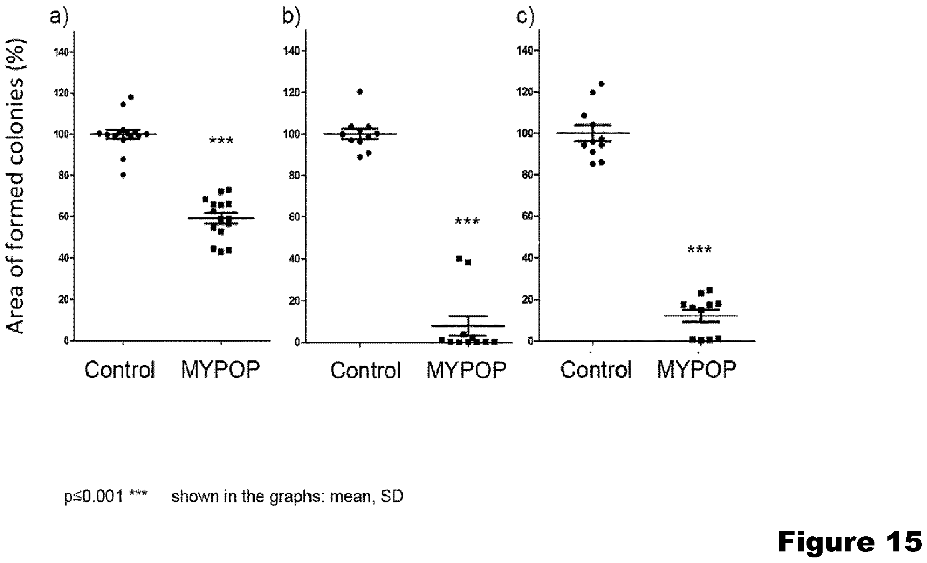

Figure 15 shows that a re-expression of MYPOP reduces cell proliferation in a) a human liver cancer cell line, b) in a lung squamous cell carcinoma cell line and c) in a lung adenocarcinoma cell line. Colony formation assay was performed as described in Figures 7 to 9. The data provide proof that cell death is induced in liver cancer cells, lung cancer cells and adenocarcinoma cancer cells.

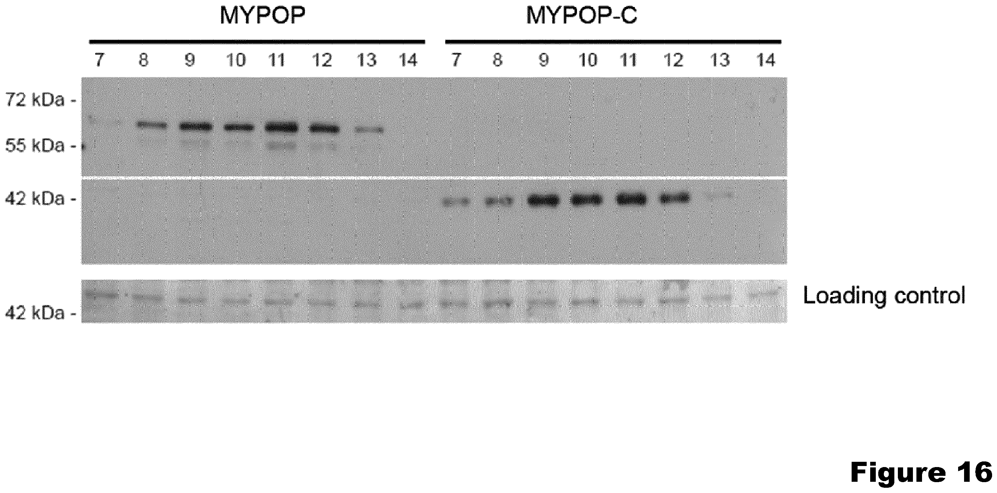

Figure 16 summarizes the results of testing of HPV16 pseudoviruses (PsVs) transduction efficiency of MYPOP and MYPOP-C PsVs by MYPOP-expression levels. MYPOP-WT or MYPOP-AD PsVs were purified and collected by Optiprep-Gradient fractionation. The Western blot assay shows MYPOP or MYPOP-C expression. Fractions 10 and 11 are peak fractions of the two gradients and were used for the subsequent experiments.

For the further experiments, PsVs were utilized as viral vectors of the MYPOP expression plasmid. Using the capsid of HPV16 as a vector for the delivery of MYPOP-Expression plasmid, pseudoviruses containing the expression plasmids of MYPOP (wild type) and MYPOP-C, a shorter version of MYPOP without the DNA binding domain, were prepared. This construct is inactive in promoter-based assays and served as control. Thereby, transfer vectors of MYPOP expression-plasmids were created. HPV16 pseudoviruses (PsVs) were produced as described previously (Buck et al., 2004). Here, MYPOP-Flag expression plasmid or the inactive deletion construct MYPOP-C-Flag was used as a pseudogenome (packaged DNA).

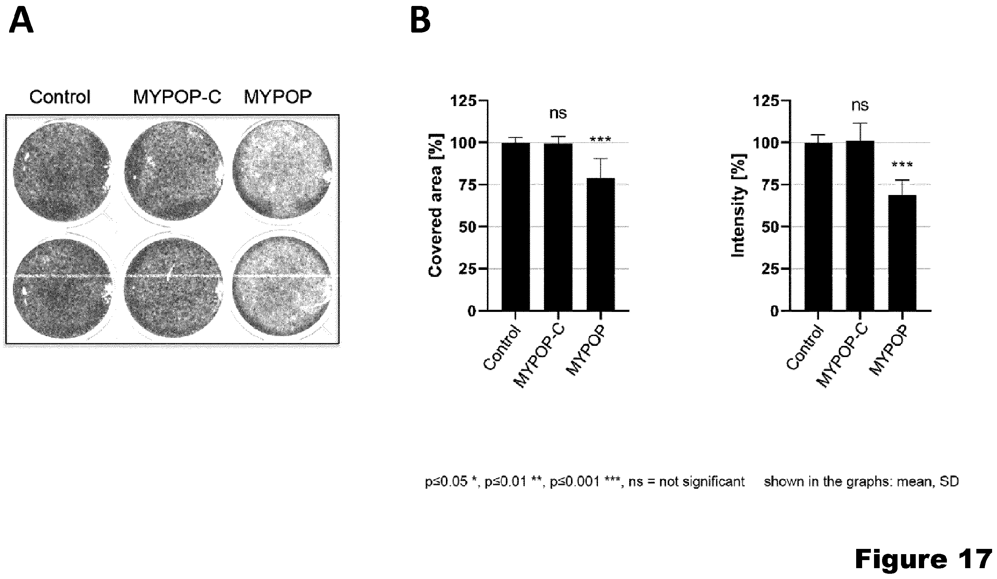

Figure 17 exemplifies that MYPOP-vector gene therapy is functional in reducing cell proliferation of keratinocyte cancer cell lines and can thus are indicative that MYPOP can be used as a beneficial therapeutic target. Colony formation assays with HPV18-transformed Hela cells were performed to test MYPOP PsVs in anticancer gene therapy. Cells were seeded and treated in regular intervals of 2 or 3 days with MYPOP PsVs versus the controls, Optiprep (control) and MYPOP-C (Figure 17 A). The killing efficiency (Figure 17B) was determined by measuring the covered area and intensity via ImageJ and Plugin "Colony area" after fixation with methanol and staining with crystal violet. A significant reduction of cell proliferation was measured after cell treatment with MYPOP-Vector. The data illustrate that MYPOP-Vector is able to reduce cell proliferation of tumor cells, making MYPOP a suitable therapeutic target for tumor therapy.

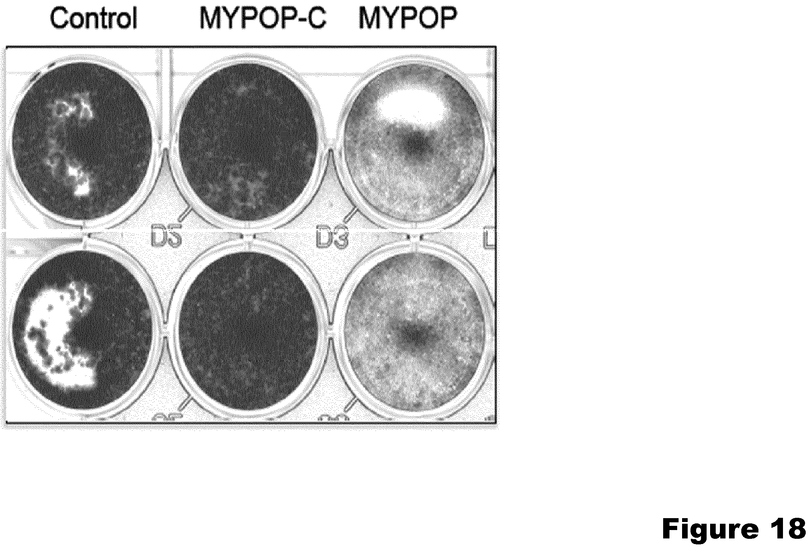

Figure 18 provides an additional example that MYPOP-vector gene therapy is functional in reducing cell proliferation of tumor cells. Here, lung cancer cell lines are treated with the MYPOP-vector. Colony formation assays with lung cancer cell lines were performed and cells were seeded and treated as in Figure 17 A.

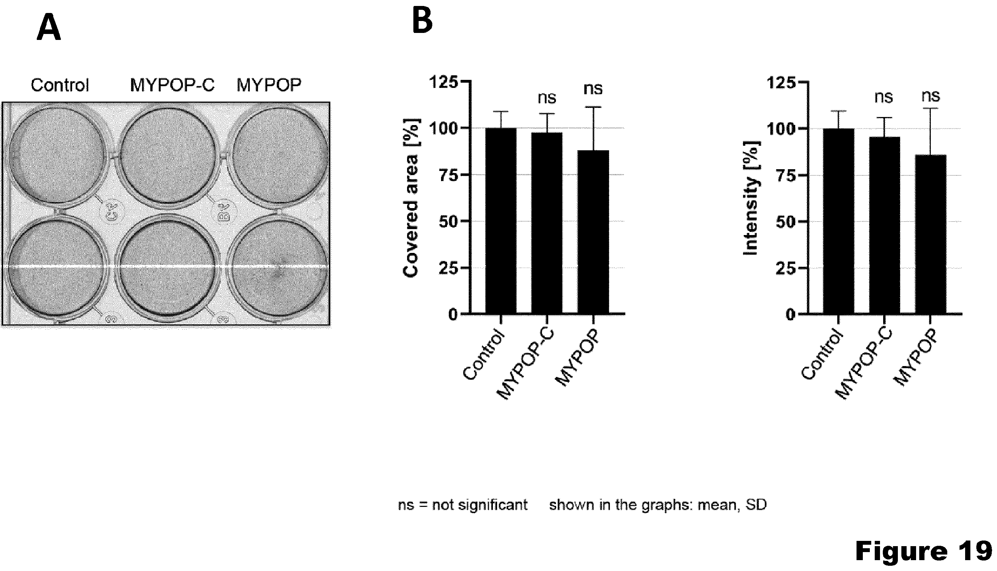

Figure 19 exemplifies that there is no significant cytotoxic effect of MYPOP-vector on normal, non-tumour cells. Colony formation assays with Normal Human Epithelial Keratinocytes (NHEK) were performed to test the putative cytotoxic side effect on normal cells (Figure 19A). Cells were seeded and treated in regular intervals of 2 or 3 days with MYPOP PsVs versus the controls, Optiprep (control) and MYPOP-C. The killing efficiency (Figure 19B) was determined by measuring the covered area and intensity via ImageJ and Plugin "Colony area" after fixation with methanol and staining with crystal violet. No significant reduction of cell proliferation was measured after cell treatment with MYPOP-Vector.

In summary, a significant decrease of cell proliferation of tumor cells can be observed by infecting cancer cells with MYPOP expressing viral vectors, whereas no effect could be observed in normal, healthy non-tumor cells. The data suggest that the invention does not only work in human but also other mammalians, and that only minor or no severe side effects are to be expected.

## Claims

1. A method for the diagnosis or prognosis of a cancer comprising
(i) determining a level of expression of Myb-related transcription factor (MYPOP) or a part thereof in a patient sample;
(ii) comparing the level of MYPOP expression of the patient sample to levels of a normal sample;
(iii) correlating the level of MYPOP expression in the patient sample relative to the levels in the normal sample with a positive or negative diagnosis or prognosis of cancer,
wherein a reduction or lack of MYPOP protein in the patient sample is indicative for a positive diagnosis of cancer.

2. The method according to claim 1, wherein the comparison of the level of protein expression of MYPOP comprises identifying a large form of MYPOP (MYPOP L form) having a molecular weight of about 60 kDa, wherein a lack of the MYPOP L form in the patient sample is indicative for a positive diagnosis of cancer.

3. The method according to claim 1, wherein the determination of the level of expression of MYPOP involves the analysis of mRNA expression or protein expression of MYPOP, a fragment of MYPOP, or truncated or elongated forms of MYPOP in the patient sample.

4. The method according to claim 1, wherein the determination of the level of expression of MYPOP protein is carried out with an antibody specific for MYPOP.

5. The method according to claim 4, wherein the determination of MYPOP protein is carried out with an antibody specific for the N-terminus of MYPOP.

6. The method according to claim 1, wherein MYPOP to be determined in the patient sample comprises a nucleic acid sequence of SEQ ID NO: 1 encoding an amino acid sequence of MYPOP, or a variant thereof in which one or more nucleic acids are substituted by degenerate nucleic acids resulting in the same amino acid sequence of MYPOP.

7. The method according to claim 1, wherein MYPOP to be determined in the patient sample comprises a nucleic acid sequence of SEQ ID NO: 1, or a homolog thereof sharing at least 75 %, at least 85 %, at least 90 % or at least 95 % homology with the nucleic acid sequence of SEQ ID NO: 1 while retaining the biological activity of MYPOP as Myb-related transcription factor.

8. The method according to claim 1, wherein MYPOP to be determined in the patient sample comprises an amino acid sequence of SEQ ID NO: 2, SEQ ID NO: 3, SEQ ID NO: 4 or SEQ ID NO: 5, or a variant thereof in which one or more amino acids are deleted, added or substituted by another amino acid while retaining the biological activity of MYPOP as Myb-related transcription factor.

9. The method according to claim 1, wherein MYPOP to be determined in the patient sample comprises an amino acid sequence of SEQ ID NO: 2, SEQ ID NO: 3, SEQ ID NO: 4 or SEQ ID NO: 5, or a or a homolog of MYPOP as found in other species sharing at least 75 %, at least 85 %, at least 90 % or at least 95 % homology with the amino acid sequence of SEQ ID NO: 2, SEQ ID NO: 3, SEQ ID NO: 4 or SEQ ID NO: 5 while retaining the biological activity of MYPOP as Myb-related transcription factor.

10. The method according to claim 1, wherein the determination of the level of expression of MYPOP is carried out by quantitatively or qualitatively analyzing the mRNA level or protein level of MYPOP using any one of the sequences according to SEQ ID NO: 1 to 5, or parts thereof.

11. A pharmaceutical composition, comprising a therapeutically effective amount of a Myb-related transcription factor (MYPOP) L form expressing molecule or MYPOP L form protein and a pharmaceutically acceptable carrier for use in the treatment or prevention of a tumor disease;
wherein the tumor disease is selected from the group consisting of melanoma, breast cancer, lung cancer, liver cancer, gastric cancer, colon cancer, pancreatic cancer, prostate cancer, ovarian cancer, lymphoma, leukemia, kidney cancer, bladder cancer, or endometrial cancer.

12. The pharmaceutical composition of claim 11, wherein MYPOP is encoded by a nucleic acid sequence of SEQ ID NO: 1, or a homolog thereof sharing at least 75 %, at least 85 %, at least 90 % or at least 95 % homology with the nucleic acid sequence of SEQ ID NO: 1 while retaining the biological activity of MYPOP as Myb-related transcription factor.

13. The pharmaceutical composition according to claim 11, wherein MYPOP comprises an amino acid sequence of SEQ ID NO: 2, SEQ ID NO: 3, SEQ ID NO: 4 or SEQ ID NO: 5, or a variant thereof in which one or more amino acids are deleted, added or substituted by another amino acid while retaining the biological activity of MYPOP as Myb-related transcription factor.

14. The pharmaceutical composition according to claim 11, wherein the MYPOP comprises an amino acid sequence of SEQ ID NO: 2, SEQ ID NO: 3, SEQ ID NO: 4 or SEQ ID NO: 5, or a or a homolog of MYPOP as found in other species sharing at least 75 %, at least 85 %, at least 90 % or at least 95 % homology with the amino acid sequence of SEQ ID NO: 3, SEQ ID NO: 4 or SEQ ID NO: 5 while retaining the biological activity of MYPOP as Myb-related transcription factor.

15. A method of enhancing cell proliferation in a cell culture comprising the incubation of the cells in a medium that contains an inhibitor of Myb-related transcription factor (MYPOP).

16. The method according to claim 15, wherein the inhibitor is an antibody.

17. The method according to claim 15, wherein the inhibitor is profilin, RNAi or siRNA.

18. The method according to claim 15, wherein the MYPOP gene is mutated or removed by MYPOP gene knockout which is introduced by e.g. CRISPR CAS9 technology.

19. The method according to claim 15, wherein the expression level of MYPOP is reduced by inhibiting protein synthesis of MYPOP, or by degrading intracellular MYPOP protein.

## Patentansprüche

1. Verfahren zur Diagnose oder Prognose eines Krebses, umfassend
(i) Bestimmen eines Expressionslevels eines Myb-verwandten Transkriptionsfaktors (MYPOP) oder eines Teils davon in einer Patientenprobe;
(ii) Vergleichen des MYPOP-Expressionslevels der Patientenprobe mit Levels einer Normalprobe;
(iii) Korrelieren des MYPOP-Expressionslevels in der Patientenprobe relativ zu den Levels in der Normalprobe mit einer positiven oder negativen Diagnose oder Prognose eines Krebses,
wobei eine Reduktion oder ein Fehlen von MYPOP-Protein in der Patientenprobe indikativ für eine positive Diagnose eines Krebses ist.

2. Verfahren nach Anspruch 1, wobei das Vergleichen des MYPOP-Proteinexpressionslevels ein Identifizieren einer großen Form von MYPOP (MYPOP-L-Form) mit einem Molekulargewicht von etwa 60 kDa umfasst, wobei ein Fehlen der MYPOP-L-Form in der Patientenprobe indikativ für eine positive Diagnose eines Krebses ist.

3. Verfahren nach Anspruch 1, wobei das Bestimmen des MYPOP-Expressionslevels die Analyse einer mRNA-Expression oder Proteinexpression eines MYPOP, eines MYPOP-Fragments, oder verkürzter oder verlängerter MYPOP-Formen in der Patientenprobe umfasst.

4. Verfahren nach Anspruch 1, wobei das Bestimmen des Expressionslevels von MYPOP-Protein mit einem für MYPOP spezifischen Antikörper durchgeführt wird.

5. Verfahren nach Anspruch 4, wobei das Bestimmen von MYPOP-Protein mit einem für den N-Terminus von MYPOP spezifischen Antikörper durchgeführt wird.

6. Verfahren nach Anspruch 1, wobei der in der Patientenprobe zu bestimmende MYPOP eine Nukleinsäuresequenz von SEQ ID NO: 1, die eine Aminosäuresequenz von MYPOP kodiert, oder eine Variante davon umfasst, bei der eine oder mehrere Nukleinsäuren durch degenerierte Nukleinsäuren ersetzt sind, resultierend in der gleichen Aminosäuresequenz von MYPOP.

7. Verfahren nach Anspruch 1, wobei der in der Patientenprobe zu bestimmende MYPOP eine Nukleinsäuresequenz von SEQ ID NO: 1 oder ein Homolog davon umfasst, das mindestens 75 %, mindestens 85 %, mindestens 90 % oder mindestens 95 % Homologie mit der Nukleinsäuresequenz von SEQ ID NO: 1 aufweist, wobei die biologische Aktivität von MYPOP als Myb-verwandter Transkriptionsfaktor erhalten bleibt.

8. Verfahren nach Anspruch 1, wobei der in der Patientenprobe zu bestimmende MYPOP eine Aminosäuresequenz von SEQ ID NO: 2, SEQ ID NO: 3, SEQ ID NO: 4 oder SEQ ID NO: 5 oder eine Variante davon umfasst, bei der eine oder mehrere Aminosäuren deletiert, hinzugefügt oder durch eine andere Aminosäure ersetzt ist/sind, wobei die biologische Aktivität von MYPOP als Myb-verwandter Transkriptionsfaktor erhalten bleibt.

9. Verfahren nach Anspruch 1, wobei der in der Patientenprobe zu bestimmende MYPOP eine Aminosäuresequenz von SEQ ID NO: 2, SEQ ID NO: 3, SEQ ID NO: 4 oder SEQ ID NO: 5 oder ein Homolog von MYPOP umfasst, wie es in anderen Spezies vorkommt, wobei es mindestens 75 %, mindestens 85 %, mindestens 90 % oder mindestens 95 % Homologie mit der Aminosäuresequenz von SEQ ID NO: 2, SEQ ID NO: 3, SEQ ID NO: 4 oder SEQ ID NO: 5 aufweist, wobei die biologische Aktivität von MYPOP als Myb-verwandter Transkriptionsfaktor erhalten bleibt.

10. Verfahren nach Anspruch 1, wobei das Bestimmen des MYPOP-Expressionslevels durch quantitatives oder qualitatives Analysieren des mRNA-Levels oder Proteinlevels von MYPOP unter Verwendung einer der Sequenzen nach SEQ ID NO: 1 bis 5, oder Teilen davon, durchgeführt wird.

11. Pharmazeutische Zusammensetzung, umfassend eine therapeutisch wirksame Menge eines Myb-verwandten Transkriptionsfaktor (MYPOP)-L-Form exprimierenden Moleküls oder eines MYPOP-L-Form-Proteins, und einen pharmazeutisch akzeptablen Träger,
zur Verwendung bei dem Behandeln oder Verhindern einer Tumorerkrankung;
wobei die Tumorerkrankung ausgewählt ist aus der Gruppe, bestehend aus Melanom, Brustkrebs, Lungenkrebs, Leberkrebs, Magenkrebs, Darmkrebs, Bauchspeicheldrüsenkrebs, Prostatakrebs, Eierstockkrebs, Lymphom, Leukämie, Nierenkrebs, Blasenkrebs oder Endometriumkrebs.

12. Pharmazeutische Zusammensetzung nach Anspruch 11, wobei MYPOP durch eine Nukleinsäuresequenz von SEQ ID NO: 1 oder ein Homolog davon kodiert ist, das mindestens 75 %, mindestens 85 %, mindestens 90 % oder mindestens 95 % Homologie mit der Nukleinsäuresequenz von SEQ ID NO: 1 aufweist, wobei die biologische Aktivität von MYPOP als Myb-verwandter Transkriptionsfaktor erhalten bleibt.

13. Pharmazeutische Zusammensetzung nach Anspruch 11, wobei MYPOP eine Aminosäuresequenz von SEQ ID NO: 2, SEQ ID NO: 3, SEQ ID NO: 4 oder SEQ ID NO: 5 oder eine Variante davon umfasst, bei der eine oder mehrere Aminosäuren deletiert, hinzugefügt oder durch eine andere Aminosäure ersetzt ist/sind, wobei die biologische Aktivität von MYPOP als Myb-verwandter Transkriptionsfaktor erhalten bleibt.

14. Pharmazeutische Zusammensetzung nach Anspruch 11, wobei der MYPOP eine Aminosäuresequenz von SEQ ID NO: 2, SEQ ID NO: 3, SEQ ID NO: 4 oder SEQ ID NO: 5 oder ein Homolog von MYPOP umfasst, wie es in anderen Spezies vorkommt, wobei es mindestens 75 %, mindestens 85 %, mindestens 90 % oder mindestens 95 % Homologie mit der Aminosäuresequenz von SEQ ID NO: 3, SEQ ID NO: 4 oder SEQ ID NO: 5 aufweist, wobei die biologische Aktivität von MYPOP als Myb-verwandter Transkriptionsfaktor erhalten bleibt.

15. Verfahren zum Steigern einer Zellproliferation in einer Zellkultur, umfassend das Inkubieren der Zellen in einem Medium, das einen Inhibitor eines Myb-verwandten Transkriptionsfaktors (MYPOP) umfasst.

16. Verfahren nach Anspruch 15, wobei der Inhibitor ein Antikörper ist.

17. Verfahren nach Anspruch 15, wobei der Inhibitor Profilin, RNAi oder siRNA ist.

18. Verfahren nach Anspruch 15, wobei das MYPOP-Gen durch einen MYPOP-Gen-Knockout, der z.B. mittels CRISPR-Cas9-Technologie eingeführt wird, mutiert oder entfernt wird.

19. Verfahren nach Anspruch 15, wobei das Expressionslevel von MYPOP durch Inhibieren einer MYPOP-Proteinsynthese oder durch Abbauen von intrazellulärem MYPOP-Protein reduziert wird.

## Revendications

1. Un procedé de diagnostic ou de pronostic d'un cancer comprenant
(i) la détermination d'un niveau d'expression du facteur de transcription apparenté à Myb (MYPOP) ou d'une partie de celui-ci dans un échantillon de patient ;
(ii) la comparaison du niveau d'expression de MYPOP de l'échantillon de patient aux niveaux d'un échantillon normal ;
(iii) la corrélation du niveau d'expression de MYPOP dans l'échantillon de patient par rapport aux niveaux dans l'échantillon normal avec un diagnostic ou un pronostic positif ou négatif de cancer,
dans lequel une patient nou une absence de protéine MYPOP dans l'échantillon de patient est indicative d'un diagnostic positif de cancer.

2. Le procedé selon la revendication 1, lequel la comparaison du niveau d'expression protéique de MYPOP comprend l'identification d'une forme L de MYPOP (forme L de MYPOP) ayant un poids moléculaire d'environ 60 kDa, lequel une absence de la forme L de MYPOP dans l'échantillon de patient est indicative d'un diagnostic positif de cancer.

3. Le procédé selon la revendication 1, lequel la détermination du niveau d'expression de MYPOP implique l'analyse de l'expression d'ARNm ou de l'expression protéique de MYPOP, d'un fragment de MYPOP, ou de formes tronquées ou allongées de MYPOP dans l'échantillon de patient.

4. Le procédé selon la revendication 1, lequel la détermination du niveau d'expression de la protéine MYPOP est réalisée avec un anticorps spécifique de MYPOP.

5. Le procédé selon la revendication 4, lequel la détermination de la protéine MYPOP est réalisée avec un anticorps spécifique de l'extrémité N-terminale de MYPOP.

6. Le procédé selon la revendication 1, lequel le MYPOP à déterminer dans l'échantillon de patient comprend une séquence d'acides nucléiques de SEQ ID NO : 1 codant une séquence d'acides aminés de MYPOP, ou une variante de celle-ci dans laquelle un ou plusieurs acides nucléiques sont substitués par des acides nucléiques dégénérés aboutissant à la même séquence d'acides aminés de MYPOP.

7. Le procédé selon la revendication 1, lequel le MYPOP à déterminer dans l'échantillon de patient comprend une séquence d'acides nucléiques de SEQ ID NO : 1, ou un homologue de celle-ci partageant au moins 75 %, au moins 85 %, au moins 90 % ou au moins 95 % d'homologie avec la séquence d'acides nucléiques de SEQ ID NO : 1 tout en conservant l'activité biologique de MYPOP en tant que facteur de transcription apparenté à Myb.

8. Le procédé selon la revendication 1, lequel le MYPOP à déterminer dans l'échantillon de patient comprend une séquence d'acides aminés de SEQ ID NO : 2, SEQ ID NO : 3, SEQ ID NO : 4 ou SEQ ID NO : 5, ou une variante de celle-ci dans laquelle un ou plusieurs acides aminés sont supprimés, ajoutés ou substitués par un autre acide aminé tout en conservant l'activité biologique de MYPOP en tant que facteur de transcription apparenté à Myb.

9. Le procédé selon la revendication 1, lequel le MYPOP à déterminer dans l'échantillon de patient comprend une séquence d'acides aminés de SEQ ID NO : 2, SEQ ID NO : 3, SEQ ID NO : 4 ou SEQ ID NO : 5, ou un homologue de MYPOP tel que trouvé dans d'autres espèces partageant au moins 75 %, au moins 85 %, au moins 90 % ou au moins 95 % d'homologie avec la séquence d'acides aminés de SEQ ID NO : 2, SEQ ID NO : 3, SEQ ID NO : 4 ou SEQ ID NO : 5 tout en conservant l'activité biologique de MYPOP en tant que facteur de transcription apparenté à Myb.

10. Le procédé selon la revendication 1, lequel la détermination du niveau d'expression de MYPOP est réalisée par analyse quantitative ou qualitative du niveau d'ARNm ou du niveau protéique de MYPOP en utilisant l'une quelconque des séquences selon SEQ ID NO : 1 à 5, ou des parties de celles-ci.

11. Une composition pharmaceutique, comprenant une quantité thérapeutiquement efficace d'une molécule exprimant la forme L du facteur de transcription apparenté à Myb (MYPOP) ou de la protéine forme L de MYPOP et un excipient pharmaceutiquement acceptable pour une utilisation dans le traitement ou la prévention d'une maladie tumorale ;
dans lequel la maladie tumorale est sélectionnée dans le groupe constitué de mélanome, cancer du sein, cancer du poumon, cancer du foie, cancer gastrique, cancer du côlon, cancer du pancréas, cancer de la prostate, cancer de l'ovaire, lymphome, leucémie, cancer du rein, cancer de la vessie ou cancer de l'endomètre.

12. La composition pharmaceutique selon la revendication 11, lequel MYPOP est codé par une séquence d'acides nucléiques de SEQ ID NO : 1, ou un homologue de celle-ci partageant au moins 75 %, au moins 85 %, au moins 90 % ou au moins 95 % d'homologie avec la séquence d'acides nucléiques de SEQ ID NO : 1 tout en conservant l'activité biologique de MYPOP en tant que facteur de transcription apparenté à Myb.

13. La composition pharmaceutique selon la revendication 11, lequel MYPOP comprend une séquence d'acides aminés de SEQ ID NO : 2, SEQ ID NO : 3, SEQ ID NO : 4 ou SEQ ID NO : 5, ou une variante de celle-ci dans laquelle un ou plusieurs acides aminés sont supprimés, ajoutés ou substitués par un autre acide aminé tout en conservant l'activité biologique de MYPOP en tant que facteur de transcription apparenté à Myb.

14. La composition pharmaceutique selon la revendication 11, lequel le MYPOP comprend une séquence d'acides aminés de SEQ ID NO : 2, SEQ ID NO : 3, SEQ ID NO : 4 ou SEQ ID NO : 5, ou un homologue de MYPOP tel que trouvé dans d'autres espèces partageant au moins 75 %, au moins 85 %, au moins 90 % ou au moins 95 % d'homologie avec la séquence d'acides aminés de SEQ ID NO : 3, SEQ ID NO : 4 ou SEQ ID NO : 5 tout en conservant l'activité biologique de MYPOP en tant que facteur de transcription apparenté à Myb.

15. Un procédé d'augmentation de la prolifération cellulaire dans une culture cellulaire comprenant l'incubation des cellules dans un milieu qui contient un inhibiteur du facteur de transcription apparenté à Myb (MYPOP).

16. Le procedé selon la revendication 15, lequel l'inhibiteur est un anticorps.

17. Le procédé selon la revendication 15, lequel l'inhibiteur est la profiline, l'ARN interférent (ARNi) ou le petit ARN interférent (siRNA).

18. Le procédé selon la revendication 15, lequel le gène MYPOP est muté ou supprimé par inactivation génique de MYPOP qui est introduite par exemple par la technologie CRISPR CAS9.

19. Le procédé selon la revendication 15, lequel le niveau d'expression de MYPOP est réduit par inhibition de la synthèse protéique de MYPOP, ou par dégradation de la protéine MYPOP intracellulaire.
